# EUROPEAN PATENT SPECIFICATION

(11) **EP 4 251 975 B1**
(45) Date of publication and mention of the grant of the patent: **22.07.2026**
(21) Application number: 21907325.1
(22) Date of filing: 15.12.2021
(51) Int. Cl.: G01N 21/64, G06K 19/06, C12Q 1/6813, C12Q 1/6874, C12Q 1/6876, C12Q 1/6869, C12Q 1/6837, C12Q 1/6841

(54) **MOLECULAR BARCODE ANALYSIS BY SINGLE-MOLECULE KINETICS**
MOLEKULARE STRICHCODEANALYSE DURCH EINZELMOLEKÜLKINETIK
ANALYSE DE CODE-BARRES MOLÉCULAIRE PAR CINÉTIQUE À MOLÉCULE UNIQUE

(30) Priority: 15.12.2020 US 202063125904 P
(43) Date of publication of application: 04.10.2023
(73) Proprietor: Quantum-Si Incorporated, Branford, CT 06405 (US)
(72) Inventor: AD, Omer, Madison, CT 06443 (US); BOER, Robert, E., Westbrook, CT 06498 (US); MCCORMACK, Evan, New Haven, CT 06511 (US); HAINING, Brianna, Leigh, San Diego, CA 92123 (US); THURSTON, Thomas, Raymond, Guilford, CT 06437 (US); REED, Brian, Madison, CT 06443 (US)
(74) Representative: Carpmaels & Ransford LLP
(86) International application number: PCT/US2021/010050
(87) International publication number: WO 2022/132188

(56) References cited:
- WO-A1-2009/065635
- WO-A1-2020/123309
- WO-A2-2016/160844
- US-A1- 2014 031 243
- US-A1- 2016 169 903
- US-B2- 10 174 363
- WADE ORSOLYA K. ET AL: "124-Color Super-resolution Imaging by Engineering DNA-PAINT Blinking Kinetics", vol. 19, no. 4, 13 March 2019 (2019-03-13), US, pages 2641 - 2646, XP055926546, ISSN: 1530-6984, Retrieved from the Internet <URL:http://pubs.acs.org/doi/pdf/10.1021/acs.nanolett.9b00508> DOI: 10.1021/acs.nanolett.9b00508
- NICOLAS CARDOZO: "Multiplexed direct detection of barcoded protein reporters on a nanopore array", BIORXIV, 11 November 2019 (2019-11-11), XP093156481, Retrieved from the Internet <URL:https://www.biorxiv.org/content/10.1101/837542v1.full.pdf> DOI: 10.1101/837542
- SAMUEL G. RODRIQUES: "A theoretical analysis of single molecule protein sequencing via weak binding spectra", PLOS ONE, vol. 14, no. 3, 28 March 2019 (2019-03-28), US, pages e0212868, XP093156437, ISSN: 1932-6203, DOI: 10.1371/journal.pone.0212868

## Description

### BACKGROUND

Advancements in microarray technologies have made it possible to conduct massively parallel analysis of single molecules in a high-throughput format. Determining the identities and origins of these molecules, and their locations within an array, is important for clinical applications, diagnostics, and biomedical research.

Wade 2019 (Nano Lett. 2019 Apr 10; 19(4):2641-2646) relates to 124-plex super-resolution imaging by engineering DNA-PAINT blinking kinetics. Cardozo 2022 (Nat Biotechnol. 2022 Jan;40(1):42-46) relates to multiplexed direct detection of barcoded protein reporters on a nanopore array. Rodriques 2019 (PLoS One. 2019 Mar 28;14(3):e0212868) relates to a theoretical analysis of single molecule protein sequencing via weak binding spectra. US 2016/169903 discloses methods for generating super-resolution patterns of molecules on substrates.

### SUMMARY

The present invention is as defined in the appended claims.

Herein, the terminologies "embodiment" and "embodiments" are to be construed as embodiment(s) of the invention only in as far as they fall within the scope of the appended claims.

In some aspects, the disclosure provides methods comprising contacting a molecular barcode with a barcode recognition molecule that binds to one or more sites on the molecular barcode. In some embodiments, the methods further comprise detecting a series of signal pulses indicative of binding interactions between the barcode recognition molecule and the molecular barcode. In some embodiments, the methods further comprise determining the identity of the molecular barcode based on a barcode-specific pattern in the series of signal pulses.

The invention provides a method comprising: contacting a molecular barcode with a barcode recognition molecule that binds to one or more sites on the molecular barcode, where the molecular barcode is attached to an analyte comprising a polypeptide; detecting a series of signal pulses indicative of binding interactions between the barcode recognition molecule and the molecular barcode; determining the identity of the molecular barcode based on a barcode-specific pattern in the series of signal pulses; and sequencing the polypeptide.

In some embodiments, the polypeptide is a protein or a protein fragment. In some embodiments, sequencing the polypeptide comprises: contacting the polypeptide with one or more terminal amino acid recognition molecules; and detecting a series of signal pulses indicative of association of the one or more terminal amino acid recognition molecules with successive amino acids exposed at a terminus of the polypeptide while the polypeptide is being degraded, thereby sequencing the polypeptide. In some embodiments, the method is performed in a single reaction vessel.

In some aspects, the disclosure provides a method comprising: contacting a molecular barcode with a barcode recognition molecule that binds to one or more sites on the molecular barcode, where the molecular barcode is attached to an analyte, and where an enzyme is bound to the analyte; detecting a series of signal pulses indicative of binding interactions between the barcode recognition molecule and the molecular barcode; and determining the identity of the molecular barcode based on a barcode-specific pattern in the series of signal pulses.

In some embodiments, the analyte is a nucleic acid. In some embodiments, the enzyme is a polymerizing enzyme. In some embodiments, the analyte is a deoxyribonucleic acid, and wherein the enzyme is a DNA polymerase. In some embodiments, the method further comprises sequencing the nucleic acid. In some embodiments, sequencing the nucleic acid comprises performing a sequencing-by-synthesis reaction whereby the enzyme synthesizes a complementary nucleic acid strand using the nucleic acid as a template. In some embodiments, the method is performed in a single reaction vessel.

In some aspects, the disclosure provides a method comprising: contacting a molecular barcode with a barcode recognition molecule that binds to one or more sites on the molecular barcode, where the molecular barcode is attached to an analyte comprising a biomolecule (e.g., a polypeptide, a nucleic acid); detecting a series of signal pulses indicative of binding interactions between the barcode recognition molecule and the molecular barcode; determining the identity of the molecular barcode based on a barcode-specific pattern in the series of signal pulses; and sequencing the biomolecule by subjecting the biomolecule to sequencing reaction conditions.

In some embodiments, the biomolecule is a polypeptide. In some embodiments, sequencing the biomolecule comprises: contacting the polypeptide with one or more terminal amino acid recognition molecules; and detecting a series of signal pulses indicative of association of the one or more terminal amino acid recognition molecules with successive amino acids exposed at a terminus of the polypeptide while the polypeptide is being degraded, thereby sequencing the polypeptide. In some embodiments, the biomolecule is a nucleic acid. In some embodiments, a polymerizing enzyme is bound to the nucleic acid. In some embodiments, the nucleic acid is a deoxyribonucleic acid, and the polymerizing enzyme is a DNA polymerase. In some embodiments, sequencing the biomolecule comprises: performing a sequencing-by-synthesis reaction whereby a polymerizing enzyme synthesizes a complementary nucleic acid strand using the nucleic acid as a template. In some embodiments, the method is performed in a single reaction vessel.

As described herein, in some embodiments, the molecular barcode is attached to an analyte. In some embodiments, the methods further comprise identifying the analyte based on a known association between the molecular barcode and the analyte. In some embodiments, identifying the analyte comprises determining the identity of a sample (e.g., a serum sample, a blood sample, a tissue sample, a single cell) from which the analyte is derived. In some embodiments, the analyte comprises a biomolecule (e.g., a polypeptide, a nucleic acid) or a fragment thereof, and identifying the analyte comprises determining the identity of the biomolecule.

In some embodiments, the molecular barcode is attached to an analyte, and the analyte is immobilized to a surface through the molecular barcode. In some embodiments, the molecular barcode is immobilized to the surface through a linkage group comprising at least one biomolecule. In some embodiments, the linkage group comprises a double-stranded nucleic acid. In some embodiments, the linkage group comprises a protein-ligand complex comprising an avidin protein bound to at least one bis-biotin moiety. In some embodiments, the linkage group comprises a double-stranded nucleic acid and a protein-ligand complex comprising an avidin protein bound to at least one bis-biotin moiety. In some embodiments, the linkage group comprises: a double-stranded nucleic acid comprising a bis-biotin moiety, wherein the double-stranded nucleic acid is attached to the molecular barcode; and an avidin protein bound to the bis-biotin moiety, wherein the avidin protein is attached to the surface.

In some embodiments, a molecular barcode of the disclosure is a nucleic acid barcode or a polypeptide barcode. In some embodiments, a barcode recognition molecule of the disclosure is an oligonucleotide. In some embodiments, the molecular barcode is a nucleic acid barcode, and the barcode recognition molecule is an oligonucleotide. In some embodiments, the oligonucleotide is at least four nucleotides in length (e.g., at least 5, at least 6, at least 7, at least 8, at least 9, or at least 10 nucleotides in length). In some embodiments, the oligonucleotide is fewer than 30 nucleotides in length (e.g., fewer than 25, fewer than 20, or fewer than 15 nucleotides in length). In some embodiments, the oligonucleotide is between about 5 and about 50 nucleotides in length (e.g., between about 5 and about 25 nucleotides in length). In some embodiments, a barcode recognition molecule of the disclosure is a protein or a nucleic acid aptamer.

In some embodiments, a barcode recognition molecule of the disclosure comprises at least one detectable label. In some embodiments, a molecular barcode of the disclosure comprises at least one detectable label. In some embodiments, the detectable label is a luminescent label or a conductivity label. In some embodiments, the detectable label is a quenched label that is unquenched during binding between the barcode recognition molecule and the molecular barcode. In some embodiments, the detectable label is an unquenched label that is quenched during binding between the barcode recognition molecule and the molecular barcode.

In some embodiments, a barcode recognition molecule of the disclosure is attached to a labeled biomolecule comprising the at least one detectable label. In some embodiments, the labeled biomolecule is a labeled nucleic acid. In some embodiments, the barcode recognition molecule is attached to the labeled biomolecule through a linkage group comprising at least one biomolecule. In some embodiments, the linkage group comprises a protein-ligand complex. In some embodiments, the protein-ligand complex comprises a multivalent protein comprising at least two ligand binding sites, where the barcode recognition molecule comprises a first ligand moiety bound to a first ligand binding site on the multivalent protein, and where the labeled biomolecule comprises a second ligand moiety bound to a second ligand binding site on the multivalent protein. In some embodiments, the multivalent protein is an avidin protein comprising four biotin binding sites, and wherein the ligand moieties are biotin moieties. In some embodiments, at least one of the biotin moieties is a bis-biotin moiety, and the bis-biotin moiety is bound to two biotin binding sites on the avidin protein.

In some embodiments, a signal pulse of the barcode-specific pattern comprises a pulse duration that is characteristic of a dissociation rate of binding between the barcode recognition molecule and a site on the molecular barcode. In some embodiments, each signal pulse of the barcode-specific pattern is separated from another by an interpulse duration that is characteristic of an association rate of barcode recognition molecule binding. In some embodiments, the series of signal pulses is a series of real-time signal pulses.

In some embodiments, the methods comprise contacting the molecular barcode with two or more barcode recognition molecules that bind to different or overlapping sites on the molecular barcode. In some embodiments, the two or more barcode recognition molecules are simultaneously contacted with the molecular barcode in the same mixture. In some embodiments, the two or more barcode recognition molecules are separately contacted with the molecular barcode in different mixtures.

In some embodiments, the methods further comprise providing a mixture comprising the molecular barcode and the barcode recognition molecule. In some embodiments, the mixture comprises a plurality of molecular barcodes, each molecular barcode of the plurality having a different analyte attached thereto. In some embodiments, the barcode recognition molecule binds two or more molecular barcodes of the plurality. In some embodiments, binding interactions between the barcode recognition molecule and each of the two or more molecular barcodes produce different barcode-specific patterns. In some embodiments, binding interactions between the barcode recognition molecule and each of the two or more molecular barcodes produces the same barcode-specific pattern. In some embodiments, the mixture comprises a plurality of barcode recognition molecules that bind to two or more molecular barcodes of the plurality.

In some embodiments, the molecular barcode comprises a series of index sequences. In some embodiments, each index sequence is different from any other index sequence of the series. In some embodiments, at least two index sequences of the series are the same. In some embodiments, the series of index sequences corresponds to a series of barcode recognition molecule binding sites. In some embodiments, the barcode recognition molecule binds to a site on the molecular barcode comprising two index sequences of the series.

In some embodiments, an analyte of the disclosure comprises a biomolecule that is derived from a biological or synthetic source. In some embodiments, the biomolecule is derived from a mixed or purified sample. In some embodiments, the biomolecule is derived from a biological sample (e.g., serum, blood, tissue, saliva, urine, or other biological source). In some embodiments, the biomolecule is derived from a synthetic library. In some embodiments, the biomolecule is obtained from a patient sample (e.g., a human sample). In some embodiments, the biomolecule is a nucleic acid or a polypeptide. In some embodiments, the biomolecule is a nucleic acid aptamer, a protein, or a protein fragment.

In some embodiments, a molecular barcode of the disclosure is attached to an analyte through a linker (e.g., a covalent or non-covalent linkage group). In some embodiments, the molecular barcode is attached to the analyte through a linker comprising a cleavage site. In some embodiments, the methods further comprise cleaving the analyte from the molecular barcode (e.g., at the cleavage site) prior to contacting the molecular barcode with a barcode recognition molecule.

In some embodiments, a molecular barcode of the disclosure is immobilized (e.g., attached) to a surface. In some embodiments, the molecular barcode is attached to the surface through a linker (e.g., a covalent or non-covalent linkage group). In some embodiments, the surface is comprised by an array. In some embodiments, the surface of the array comprises a plurality of molecular barcodes attached thereto.

In some embodiments, a plurality of barcode recognition processes in accordance with the disclosure are performed in parallel on an array. In some embodiments, the array comprises an array of sample wells. In some embodiments, an array comprises between about 10,000 and about 1,000,000 sample wells. In some embodiments, the volume of a sample well is between about 10⁻²¹ liters and about 10⁻¹⁵ liters.

In some aspects, the disclosure provides systems comprising at least one hardware processor, and at least one non-transitory computer-readable storage medium storing processor-executable instructions that, when executed by the at least one hardware processor, cause the at least one hardware processor to perform a method in accordance with the disclosure. In some aspects, the disclosure provides at least one non-transitory computer-readable storage medium storing processor-executable instructions that, when executed by at least one hardware processor, cause the at least one hardware processor to perform a method in accordance with the disclosure.

The details of certain embodiments of the invention are set forth in the Detailed Description, as described below. Other features, objects, and advantages of the invention will be apparent from the Examples, Drawings, and Claims.

### BRIEF DESCRIPTION OF THE DRAWINGS

The accompanying drawings, which constitute a part of this specification, illustrate several embodiments of the invention and together with the description, serve to explain the principles of the invention.
**FIG. 1** shows an example of molecular barcode recognition by detection of single-molecule binding interactions.
**FIG. 2** shows an example of a dynamic peptide sequencing reaction by detection of single-molecule binding interactions.
**FIG. 3** shows an example of signal pulse detection and analysis.
**FIG. 4** shows an example of a molecular barcode construct for use in accordance with embodiments of the disclosure.
**FIGs. 5A-5B** show an example of barcode recognition used in connection with single-cell polypeptide sequencing. FIG. 5A shows a general process in which polypeptides from single cells are labeled with cell-specific barcodes. FIG. 5B generically depicts barcoded polypeptides, which can be analyzed by dynamic sequencing and barcode recognition on a single array substrate.
**FIG. 6** shows an example schematic of a pixel of an integrated device.

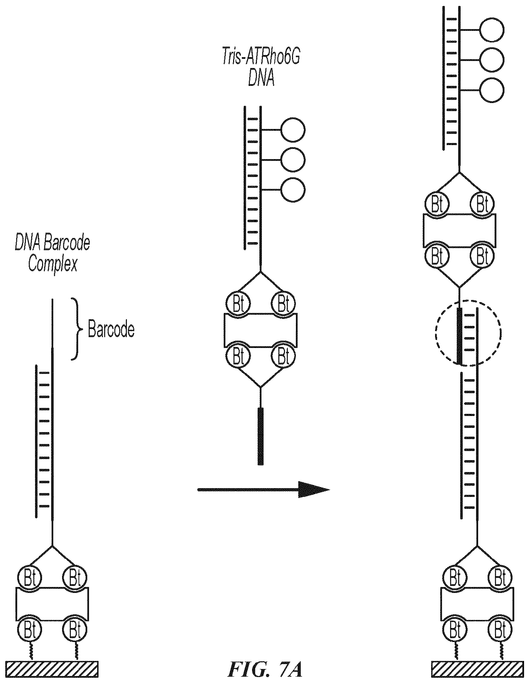

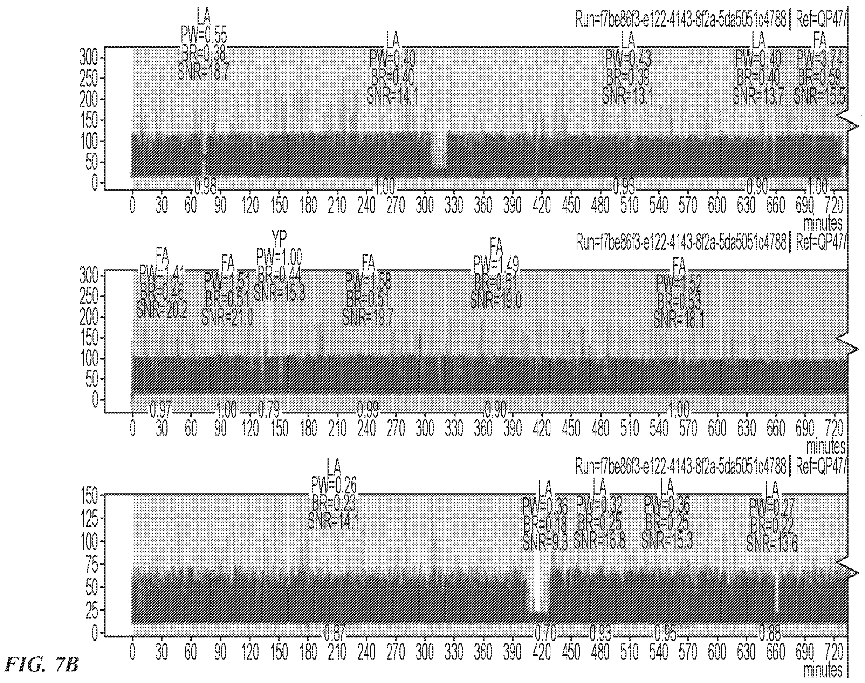

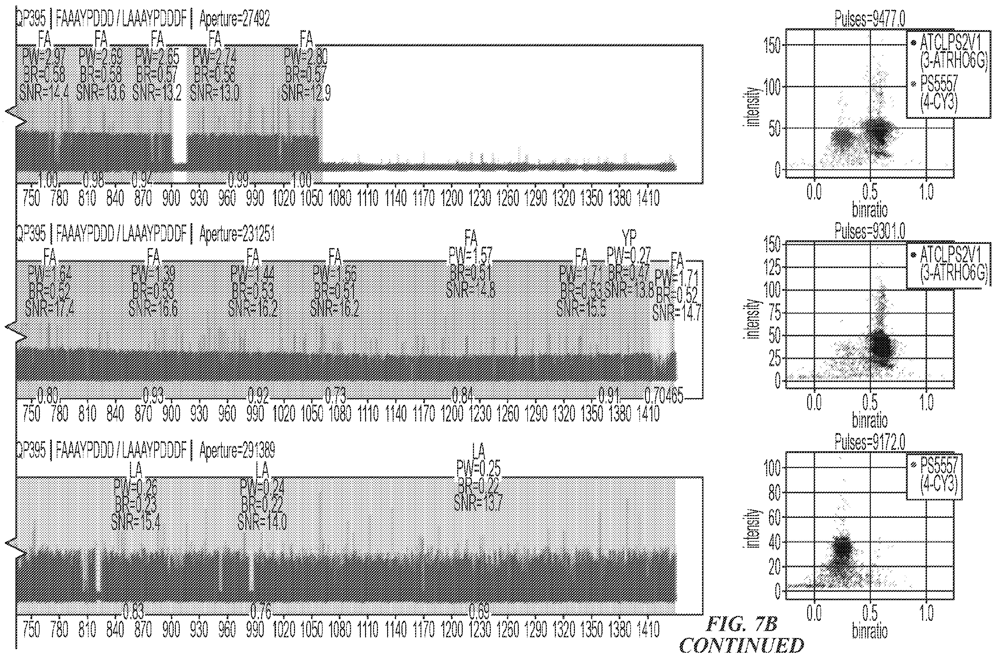

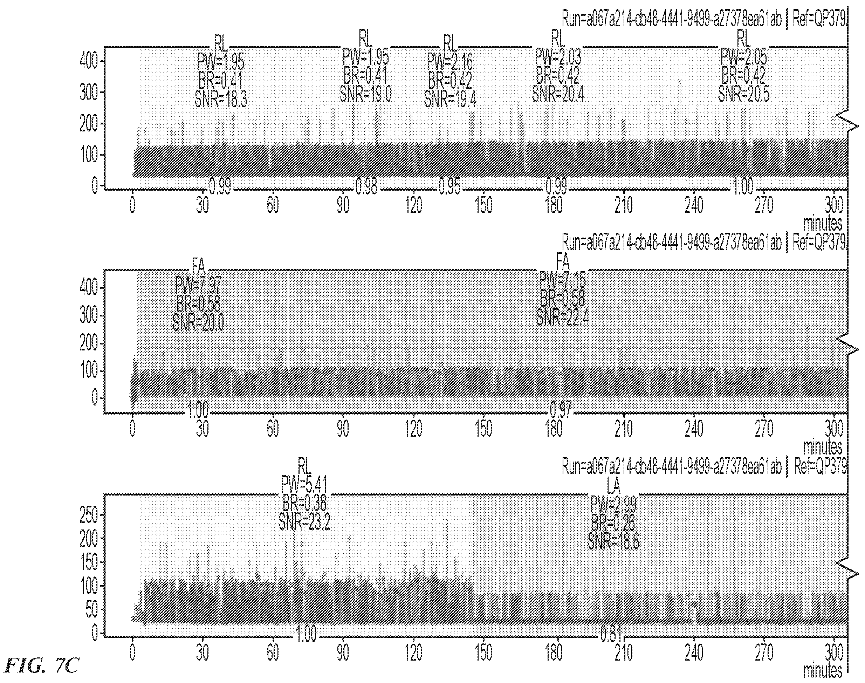

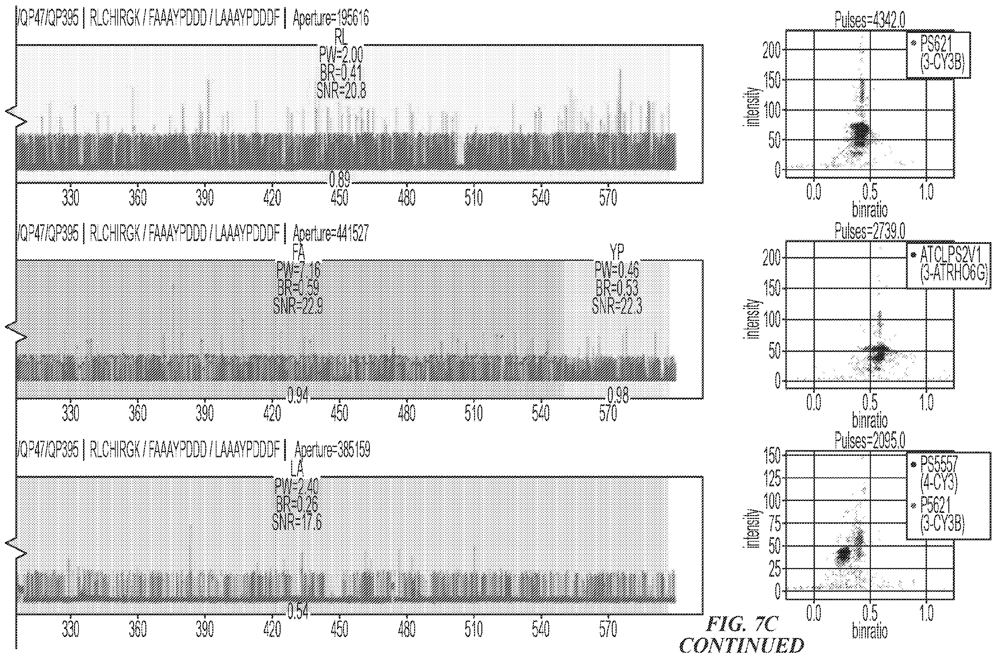

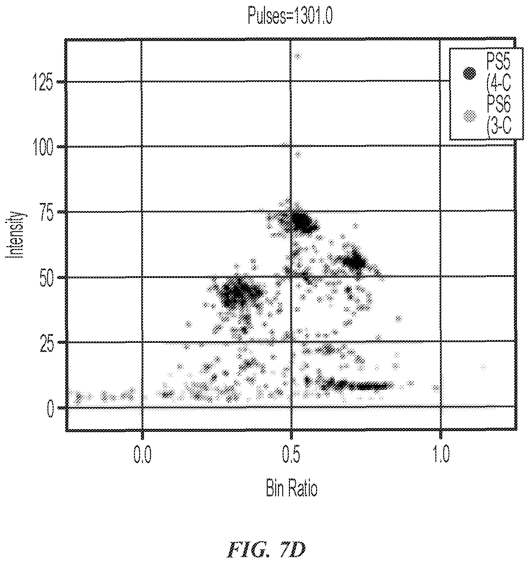

**FIGs. 7A-7D** show an example of barcode recognition by oligonucleotide hybridization. FIG. 7A shows an illustration of a process for barcode recognition by contacting a DNA barcode complex with an oligonucleotide probe that hybridizes to the DNA barcode. FIG. 7B shows single molecule intensity traces which illustrate hybridization of oligonucleotide probes to two different barcodes in a single reaction chamber. FIG. 7C shows single molecule intensity traces which illustrate hybridization of oligonucleotide probes to three different barcodes in a single reaction chamber. FIG. 7D is a plot showing that three different barcodes can be distinguished from one another based on intensity and lifetime data.
**FIG. 8** shows an example illustration of a process for barcode recognition and peptide sequencing.
**FIGs. 9A-9C** show data obtained in single molecule experiments involving barcode recognition and amino acid recognition in a single reaction chamber. FIG. 9A shows lifetime measurements determined during an assay of barcode recognition (left plot) and an assay of amino acid recognition (right plot). FIG. 9B shows lifetime measurements determined during a combined assay of barcode and amino acid recognition. FIG. 9C is a plot showing distribution of lifetime measurements (bin ratios) determined in the individual and combined assays of FIGs. 9A-9B.
**FIGs. 10A-10G** show data obtained in single molecule experiments involving barcode recognition and peptide sequencing in a single reaction chamber. FIGs. 10A-10D show lifetime measurements determined during an assay of barcode and amino acid recognition using a single barcoded polypeptide. FIGs. 10E-10F show lifetime measurements determined during an assay of barcode and amino acid recognition using two different barcoded polypeptides. FIG. 10G shows that the addition of a cleaving reagent, which removes N-terminal amino acids, eliminates amino acid recognition.
**FIG. 11** shows data obtained in single molecule experiments involving barcode recognition of two different barcodes, illustrating that different kinetic pulse properties can be used to differentiate one barcode from another.
**FIGs. 12A-12E** show an example of barcode recognition via hybridization. FIG. 12A generically depicts combinatorial barcodes, which can be produced by ligation of index sequences. FIG. 12B shows an example illustration of barcode recognition using oligonucleotide probes of different lengths. FIG. 12C shows an example workflow for a barcode recognition assay. FIG. 12D shows on-chip imaging of recognition assays performed in parallel (region highlighted in top image shown zoomed in bottom image). FIG. 12E shows plots evaluating binding frequency (top) and τₒₙ (bottom).
**FIGs. 13A-13C** show an example of barcode recognition used in connection with a single-molecule screening assay. FIG. 13A shows an example coding construct and resulting product from *in vitro* transcription/translation. FIG. 13B shows signal traces for barcode recognition (top) and antibody/antigen screening (bottom). FIG. 13C shows an example workflow for a directed evolution screening approach.

### DETAILED DESCRIPTION

Aspects of the disclosure relate to methods of molecular barcode recognition by detecting single-molecule binding interactions, and compositions for performing such methods. In some aspects, methods of the disclosure provide an approach for deconvoluting molecular barcode content from a multiplexed sample based on kinetic information corresponding to single-molecule binding interactions at one or more sites on a molecular barcode.

In some aspects, the disclosure relates to the discovery of molecular barcoding techniques which leverage conventional barcoding principles in conjunction with advancements in single-molecule analysis that allow for discrete binding events to be monitored in real-time. The inventors have recognized and appreciated that molecular barcode content can be interrogated using probes to evaluate content-specific binding kinetics, which provides an alternative or additional dimension to conventional barcode analysis.

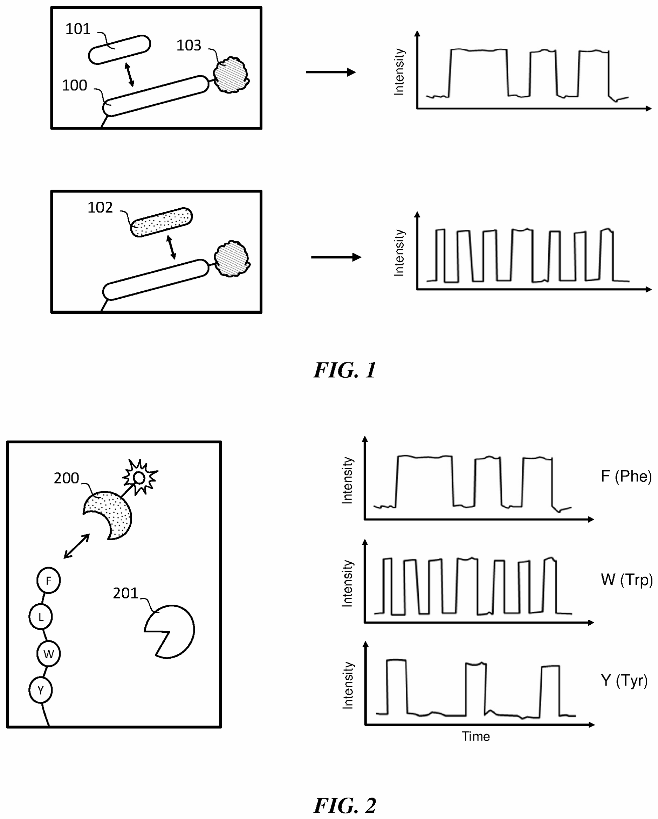

In some aspects, methods of the disclosure relate to barcode recognition by monitoring single-molecule binding interactions in real-time. FIG. 1 shows an example of barcode recognition in accordance with embodiments described herein. In some embodiments, a molecular barcode 100 is contacted with at least one barcode recognition molecule that binds to and dissociates from one or more sites on the molecular barcode. In some embodiments, different barcode content information can be obtained based on different binding interactions. For example, in some embodiments, different binding interactions can be observed for different barcode recognition molecules binding to the same or different barcode sites. In some embodiments, different binding interactions can be observed for the same barcode recognition molecule binding to different barcode sites.

As shown in the top panels, in some embodiments, the molecular barcode 100 is contacted with a first barcode recognition molecule 101 that binds to a first site on the molecular barcode 100, which produces a first pattern in signal pulse data. As shown in the bottom panels, in some embodiments, the molecular barcode 100 is contacted with a second barcode recognition molecule 102 that binds to a second site on the molecular barcode 100, which produces a second pattern in signal pulse data. In some embodiments, each of the different binding interactions produces a different pattern in signal pulse data. As described elsewhere herein, these different patterns in signal pulse data can be used to determine different content information about the molecular barcode 100. In some embodiments, the molecular barcode 100 is attached to analyte 103, and the molecular barcode content is associated with information about analyte 103.

It should be appreciated that, in some embodiments, binding interactions are a factor of both the chemical composition of a barcode recognition molecule and the site on a barcode to which it binds. For example, in some embodiments, a molecular barcode is a nucleic acid barcode, and a barcode recognition molecule is an oligonucleotide probe. Where the oligonucleotide probe binds to a site on the nucleic acid barcode, a single base modification of the oligonucleotide probe will not necessarily eliminate its ability to bind the site - rather, this modification will likely alter the binding kinetics observed between the oligonucleotide probe and the site (e.g., producing different binding profiles, as illustrated in the signal traces of FIG. 1). Likewise, where a particular oligonucleotide probe binds to two or more nucleic acid barcode sites that are chemically distinct (e.g., differing by a single base), different binding kinetics will be observed between the oligonucleotide at each of the different barcode sites. Accordingly, in some aspects, methods of the disclosure are highly sensitive, require fewer reagents, and are able to be engineered to achieve a desired result.

In some aspects, methods described herein follow similar principles as a dynamic peptide sequencing reaction. Accordingly, in some aspects, the disclosure relates to the discovery of techniques that allow for polypeptide sequencing and molecular barcode recognition to be performed simultaneously (e.g., in the same reaction mixture) or on a single surface (e.g., in a single reaction vessel). As these techniques follow similar principles throughout, this streamlines data analysis to provide a more efficient and inexpensive overall process for sequencing and barcode analysis.

By way of background, dynamic peptide sequencing reactions are carried out in real-time by evaluating binding interactions between amino acid recognition molecules and a terminal end of a polypeptide as amino acids are progressively cleaved from the terminal end. FIG. 2 shows an example of a dynamic peptide sequencing reaction in which discrete binding events give rise to signal pulses of a signal output. The inset panel (left) of FIG. 2 illustrates a general scheme of dynamic peptide sequencing. As shown, an amino acid recognition molecule 200 and a cleaving reagent 201 are present in a sequencing reaction mixture with a polypeptide of interest. The amino acid recognition molecule 200 associates with (e.g., binds to) and dissociates from a terminal amino acid, and a detectable signal is produced for the duration of each association event. As this on-off binding generally occurs at a faster rate than amino acid cleavage by the cleaving reagent 201, the binding events give rise to a series of pulses in a signal output which may be used to identify a particular terminal amino acid.

FIG. 2 shows the progress of signal output intensity over time (right panels) for the example polypeptide shown in the inset panel (left). As generally depicted, binding events involving one type of terminal amino acid will produce a characteristic pattern in the series of pulses that is distinguishable from characteristic patterns observed for other types of terminal amino acids. By monitoring these events in real-time, signal pulse data can be analyzed to determine a series of characteristic patterns corresponding to amino acid sequence information for the polypeptide. Methods and compositions for performing dynamic sequencing and analyzing data obtained therefrom are described more fully in PCT International Publication No. WO2020102741A1, filed November 15, 2019, titled "METHODS AND COMPOSITIONS FOR PROTEIN SEQUENCING," and PCT International Publication No. WO2021236983A2, filed May 20, 2021, titled "METHODS AND COMPOSITIONS FOR PROTEIN SEQUENCING".

### Single-Molecule Kinetics

Aspects of the disclosure relate to identifying content of a molecular barcode. As used herein, "identifying," "recognizing," "recognition," and like terms, in reference to a molecular barcode includes determination of partial identity (e.g., partial sequence information) as well as full identity (e.g., full sequence information) of the molecular barcode. In some embodiments, the terminology includes determining or inferring a nucleotide sequence of at least a portion of a molecular barcode (e.g., based on complementarity with an oligonucleotide probe). In yet other embodiments, the terminology includes determining or inferring certain characteristics of a molecular barcode, such as the presence or absence of a particular index sequence at one or more sites on a molecular barcode. Accordingly, in some embodiments, the terms "barcode content," "barcode identity," and like terms as used herein may refer to qualitative information pertaining to a molecular barcode and are not restricted to the specific sequence information (e.g., the nucleotide sequence of an index) that biochemically characterizes a molecular barcode.

In some embodiments, barcode recognition is performed by observing different association events between a barcode recognition molecule and a molecular barcode, where each association event produces a change in magnitude of a signal that persists for a duration of time. In some embodiments, these changes in magnitude are detected as a series of signal pulses, or a series of pulses in a signal trace output.

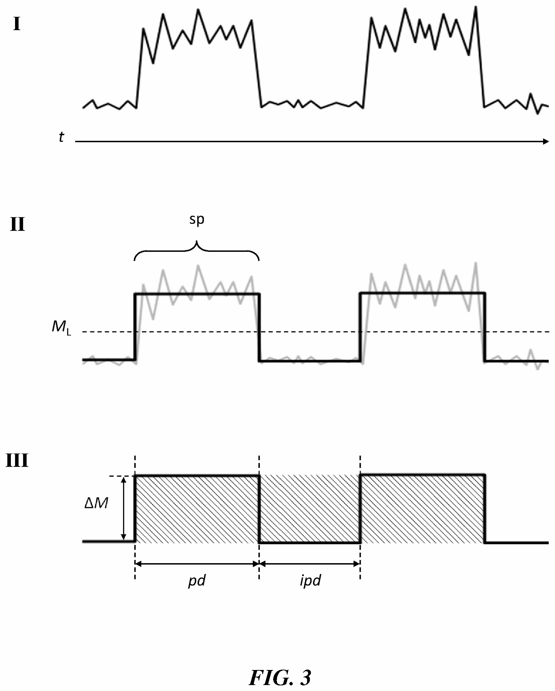

A non-limiting example of signal trace output and analysis is shown in FIG. 3. An example signal trace (I) is depicted with two signal pulses which each manifest as a peak in signal intensity that persists for a duration of time corresponding to an association event. Accordingly, the time duration between the two signal pulses having an approximately baseline signal may correspond to a duration of time during which a molecular barcode is not detectably associated with a barcode recognition molecule. In some embodiments, signal pulse data can be analyzed as illustrated in panels (II) and (III).

In some embodiments, signal data can be analyzed to extract signal pulse information by applying threshold levels to one or more parameters of the signal data. For example, panel (II) depicts a threshold magnitude level ("*M*_{L}") applied to the signal data of the example signal trace (I). In some embodiments, *M*_{L} is a minimum difference between a signal detected at a point in time and a baseline determined for a given set of data. In some embodiments, a signal pulse ("sp") is assigned to each portion of the data that is indicative of a change in magnitude exceeding *M*_{L} and persisting for a duration of time. In some embodiments, a threshold time duration may be applied to a portion of the data that satisfies *M*_{L} to determine whether a signal pulse is assigned to that portion. For example, experimental artifacts may give rise to a change in magnitude exceeding *M*_{L} that does not persist for a duration of time sufficient to assign a signal pulse with a desired confidence (e.g., non-specific detection events such as diffusion into an observation region or reagent sticking within an observation region). Accordingly, in some embodiments, a signal pulse is extracted from signal data based on a threshold magnitude level and a threshold time duration.

Extracted signal pulse information is shown in panel (II) with the example signal trace (I) superimposed for illustrative purposes. In some embodiments, a peak in magnitude of a signal pulse is determined by averaging the magnitude detected over a duration of time that persists above *M*_{L}. It should be appreciated that, in some embodiments, a "signal pulse" as used herein can refer to a change in signal data that persists for a duration of time above a baseline (e.g., raw signal data, as illustrated by the example signal trace (I)), or to signal pulse information extracted therefrom (e.g., processed signal data, as illustrated in panel (III)).

Panel (III) shows the signal pulse information extracted from the example signal trace (I). As shown, each signal pulse comprises a pulse duration *("pd")* corresponding to an association event between a barcode recognition molecule and a molecular barcode. In some embodiments, the pulse duration is characteristic of a dissociation rate of binding. Also as shown, each signal pulse is separated from another signal pulse by an interpulse duration *("ipd").* In some embodiments, the interpulse duration is characteristic of an association rate of binding. In some embodiments, a change in magnitude *("ΔM')* can be determined for a signal pulse based on a difference between baseline and the peak of a signal pulse.

In some embodiments, signal pulse information can be analyzed to identify barcode content based on a barcode-specific pattern in a series of signal pulses. For example, as shown in panel (III), in some embodiments, a barcode-specific pattern (shaded region) is determined based on pulse duration and interpulse duration. In some embodiments, a barcode-specific pattern is determined based on pulse duration, or a summary statistic for pulse duration as described elsewhere herein. In some embodiments, a barcode-specific pattern is determined based on any one or more of pulse duration, interpulse duration, and change in magnitude. In some embodiments, a barcode-specific pattern is determined to be associated with a particular feature and/or sequence of a molecular barcode (e.g., barcode content) based on reference data.

Accordingly, as illustrated by FIG. 3, in some embodiments, methods of the disclosure are performed by detecting a series of signal pulses indicative of association (e.g., binding) of a barcode recognition molecule with a molecular barcode. The series of signal pulses can be analyzed to determine a barcode-specific pattern in the series of signal pulses, and the barcode-specific pattern can be used to decipher barcode content.

In some embodiments, the series of signal pulses comprises a series of changes in magnitude of an optical signal over time. In some embodiments, the series of changes in the optical signal comprises a series of changes in luminescence produced during association events. In some embodiments, luminescence is produced by a detectable label associated with one or more reagents for barcode recognition. For example, in some embodiments, a barcode recognition molecule comprises a luminescent label. Examples of luminescent labels and their use in accordance with the disclosure are provided elsewhere herein.

In some embodiments, the series of signal pulses comprises a series of changes in magnitude of an electrical signal over time. In some embodiments, the series of changes in the electrical signal comprises a series of changes in conductance produced during association events. In some embodiments, conductivity is produced by a detectable label associated with one or more reagents for barcode recognition. For example, in some embodiments, a barcode recognition molecule comprises a conductivity label. Methods for identifying single molecules using conductivity labels have been described (see, e.g., U.S. Patent Publication No. 2017/0037462).

As described herein, signal pulse information may be used to identify barcode content based on a barcode-specific pattern in a series of signal pulses. In some embodiments, a barcode-specific pattern comprises a plurality of signal pulses, each signal pulse comprising a pulse duration. In some embodiments, the plurality of signal pulses may be characterized by a summary statistic (e.g., mean, median, time decay constant) of the distribution of pulse durations in a barcode-specific pattern. In some embodiments, the mean pulse duration of a barcode-specific pattern is between about 1 millisecond and about 10 seconds (e.g., between about 1 ms and about 1 s, between about 1 ms and about 100 ms, between about 1 ms and about 10 ms, between about 10 ms and about 10 s, between about 100 ms and about 10 s, between about 1 s and about 10 s, between about 10 ms and about 100 ms, or between about 100 ms and about 500 ms). In some embodiments, the mean pulse duration is between about 50 milliseconds and about 2 seconds, between about 50 milliseconds and about 500 milliseconds, or between about 500 milliseconds and about 2 seconds.

In some embodiments, different barcode-specific patterns corresponding to different barcode content may be distinguished from one another based on a statistically significant difference in the summary statistic. For example, in some embodiments, one barcode-specific pattern may be distinguishable from another barcode-specific pattern based on a difference in mean pulse duration of at least 10 milliseconds (e.g., between about 10 ms and about 10 s, between about 10 ms and about 1 s, between about 10 ms and about 100 ms, between about 100 ms and about 10 s, between about 1 s and about 10 s, or between about 100 ms and about 1 s). In some embodiments, the difference in mean pulse duration is at least 50 ms, at least 100 ms, at least 250 ms, at least 500 ms, or more. In some embodiments, the difference in mean pulse duration is between about 50 ms and about 1 s, between about 50 ms and about 500 ms, between about 50 ms and about 250 ms, between about 100 ms and about 500 ms, between about 250 ms and about 500 ms, or between about 500 ms and about 1 s. In some embodiments, the mean pulse duration of one barcode-specific pattern is different from the mean pulse duration of another barcode-specific pattern by about 10-25%, 25-50%, 50-75%, 75-100%, or more than 100%, for example by about 2-fold, 3-fold, 4-fold, 5-fold, or more. It should be appreciated that, in some embodiments, smaller differences in mean pulse duration between different barcode-specific patterns may require a greater number of pulse durations within each barcode-specific pattern to distinguish one from another with statistical confidence.

In some embodiments, a barcode-specific pattern generally refers to a plurality of association (e.g., binding) events between a barcode recognition molecule and a molecular barcode. In some embodiments, a barcode-specific pattern comprises at least 10 association events (e.g., at least 25, at least 50, at least 75, at least 100, at least 250, at least 500, at least 1,000, or more, association events). In some embodiments, a barcode-specific pattern comprises between about 10 and about 1,000 association events (e.g., between about 10 and about 500 association events, between about 10 and about 250 association events, between about 10 and about 100 association events, or between about 50 and about 500 association events). In some embodiments, the plurality of association events is detected as a plurality of signal pulses.

In some embodiments, a barcode-specific pattern refers to a plurality of signal pulses which may be characterized by a summary statistic as described herein. In some embodiments, a barcode-specific pattern comprises at least 10 signal pulses (e.g., at least 25, at least 50, at least 75, at least 100, at least 250, at least 500, at least 1,000, or more, signal pulses). In some embodiments, a barcode-specific pattern comprises between about 10 and about 1,000 signal pulses (e.g., between about 10 and about 500 signal pulses, between about 10 and about 250 signal pulses, between about 10 and about 100 signal pulses, or between about 50 and about 500 signal pulses).

In some embodiments, a barcode-specific pattern refers to a plurality of association (e.g., binding) events between a barcode recognition molecule and a molecular barcode occurring over a time interval. In some embodiments, barcode recognition may be carried out by iterative wash cycles in which molecular barcodes are exposed to different sets of barcode recognition molecules over different time durations. In some embodiments, the time interval of a barcode-specific pattern is between about 1 minute and about 30 minutes (e.g., between about 1 minute and about 20 minutes, between about 1 minute and 10 minutes, between about 5 minutes and about 20 minutes, between about 5 minutes and about 15 minutes, or between about 5 minutes and about 10 minutes).

In some embodiments, experimental conditions can be configured to achieve a time interval that allows for sufficient association events which provide a desired confidence level with a barcode-specific pattern (e.g., before a given set of barcode recognition molecules is removed during wash cycles). This can be achieved, for example, by configuring the reaction conditions based on various properties, including: reagent concentration, molar ratio of one reagent to another (e.g., ratio of barcode recognition molecule to molecular barcode, ratio of one barcode recognition molecule to another), number of different reagent types (e.g., the number of different types of barcode recognition molecules), binding properties (e.g., kinetic and/or thermodynamic binding parameters for barcode recognition molecule binding), reagent modification (e.g., polyol and other protein modifications which can alter interaction dynamics), reaction mixture components (e.g., one or more components, such as pH, buffering agent, salt, divalent cation, surfactant, and other reaction mixture components described herein), temperature of the reaction, and various other parameters apparent to those skilled in the art, and combinations thereof. The reaction conditions can be configured based on one or more aspects described herein, including, for example, signal pulse information (e.g., pulse duration, interpulse duration, change in magnitude), labeling strategies (e.g., number and/or type of fluorophore, linkage groups), surface modification (e.g., modification of sample well surface, including molecular barcode immobilization), sample preparation (e.g., analyte size, molecular barcode modification for immobilization), and other aspects described herein.

### Molecular Barcodes

In some embodiments, methods provided herein comprise contacting a molecular barcode with a barcode recognition molecule that binds one or more sites on the molecular barcode. In some embodiments, a barcode recognition molecule binds one or more sites on a plurality of molecular barcodes. Accordingly, in some embodiments, a barcode recognition molecule can be used to decipher barcode content from a plurality of different single molecules in a mixture (e.g., different analytes comprising the same or different molecular barcodes). As an illustrative and non-limiting example, a multiplexed mixture can include a plurality of analytes attached to molecular barcodes. Some of these molecular barcodes can include a sample index that is indicative of sample origin for the analyte attached thereto, and a barcode recognition molecule that binds the sample index can be used to determine which analytes originated from the corresponding sample.

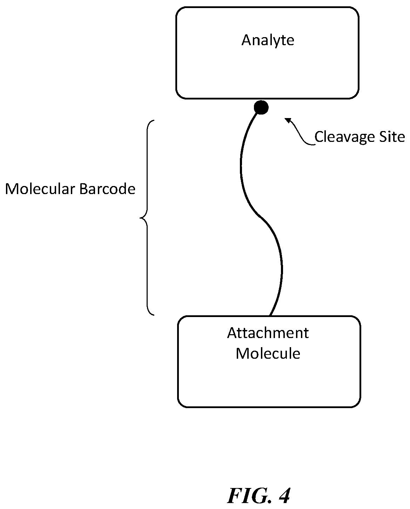

In some embodiments, a single-molecule construct for use in the methods of the disclosure may be of a general form as shown in FIG. 4. In some embodiments, the single-molecule construct includes a molecular barcode (e.g., kinetic barcode). In some embodiments, a molecular barcode of the disclosure is a nucleic acid barcode (e.g., a single-stranded nucleic acid). In some embodiments, a nucleic acid barcode comprises DNA, RNA, PNA, and/or LNA. In some embodiments, a molecular barcode is a polypeptide barcode.

In some embodiments, a molecular barcode comprises a series of index sequences. For example, in some embodiments, a molecular barcode is a nucleic acid barcode comprising a series of index sequences. In some embodiments, each index sequence is different from any other index sequence of the series. In some embodiments, at least two index sequences of the series are the same. In some embodiments, the series of index sequences corresponds to a series of barcode recognition molecule binding sites. In some embodiments, a barcode recognition molecule binds to a site on the molecular barcode comprising two index sequences of the series. In some embodiments, each index sequence provides different information with respect to barcode content.

As further shown in FIG. 4, in some embodiments, a molecular barcode is attached to an analyte (e.g., a payload molecule, a detector molecule). In some embodiments, an analyte is derived from a biological or synthetic source. In some embodiments, an analyte is derived from a serum sample, a blood sample, a tissue sample, or a single cell. In some embodiments, an analyte is a biomolecule. In some embodiments, an analyte is a nucleic acid or a polypeptide. In some embodiments, an analyte is a nucleic acid aptamer, a protein, or a protein fragment. In some embodiments, an analyte is a small molecule, a metabolite, or an antibody. In some embodiments, a molecular barcode is attached to an analyte via a linker. In some embodiments, the linker comprises a cleavage site (e.g., a photocleavable site). Accordingly, in some embodiments, a single-molecule construct comprising a cleavage sequence would allow for the removal of the analyte to simplify loading and/or analysis on a substrate surface (e.g., a chip).

Also as shown in FIG. 4, in some embodiments, a molecular barcode comprises an attachment molecule. In some embodiments, an attachment molecule is any moiety or linkage group suitable for surface immobilization of the molecular barcode. In some embodiments, the attachment molecule comprises a covalent or non-covalent linkage group. In some embodiments, the attachment molecule comprises a biotin moiety. In some embodiments, the attachment molecule comprises a bis-biotin moiety. Linkage groups and other compositions and methods useful for surface immobilization are described in further detail elsewhere herein and are known in the art.

It should be appreciated that FIG. 4 provides but one example configuration and is non-limiting with respect to single-molecule constructs of the disclosure. For example, in some embodiments, a cleavage site is an optional component which may not be incorporated into a single-molecule construct depending on a desired implementation. In some embodiments, again referring to FIG. 4, an attachment molecule can be adjacent to an analyte, such that a molecular barcode may be attached to a surface through the analyte. Examples of other configurations of single-molecule constructs and linkage strategies are provided elsewhere herein.

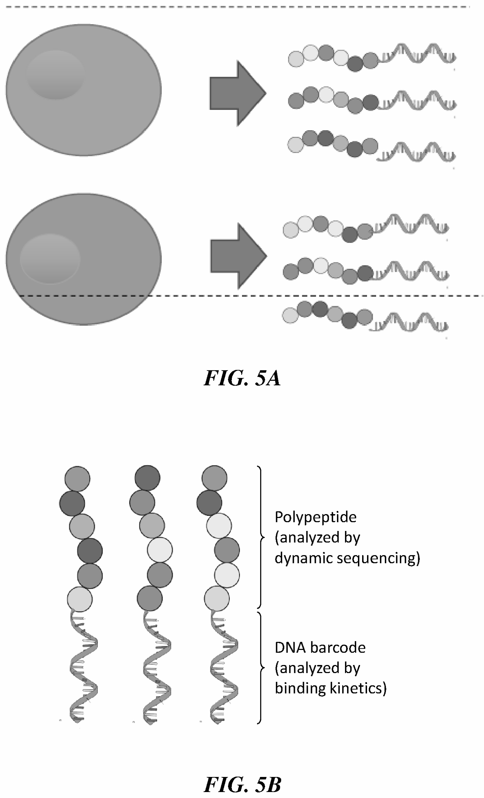

In some aspects, methods of the disclosure relate to a barcode deconvolution approach that involves deciphering molecular identity, sample origin, and/or location of a single molecule on an array. In some embodiments, methods provided herein are advantageously used to deconvolute molecular barcode information in a multiplexed sample. For example, methods of the disclosure can be applied to techniques for single-cell polypeptide sequencing. FIG. 5A shows a general process in which polypeptide molecules from single cells are labeled with cell-specific barcodes. In some embodiments, the resulting single-molecule constructs can be analyzed by polypeptide sequencing (e.g., dynamic peptide sequencing) and barcode recognition in accordance with the disclosure (FIG. 5B).

### Barcode Recognition Molecules

In some aspects, the disclosure provides barcode recognition molecules and methods of using the same. In some embodiments, a barcode recognition molecule can be selected or engineered based on desired binding kinetics with respect to a barcode site. For example, in some aspects, methods described herein can be performed in a multiplexed format in which a plurality of sites must be distinguished from one another based on binding interactions at each site. As such, the binding interactions at one site should be sufficiently different from binding interactions at another site, such that the different sites can be distinguished with higher confidence based on signal pulse information.

Without wishing to be bound by theory, a barcode recognition molecule binds to a barcode site according to a binding affinity (K_{D}) defined by an association rate, or an "on" rate, of binding (kₒₙ) and a dissociation rate, or an "off" rate, of binding (k_{off}). The rate constants k_{off} and kₒₙ are the critical determinants of pulse duration (e.g., the time corresponding to a detectable association event) and interpulse duration (e.g., the time between detectable association events), respectively. In some embodiments, these kinetic rate constants can be engineered to achieve pulse durations and pulse rates (e.g., the frequency of signal pulses) that give the best accuracy.

In some embodiments, a barcode recognition molecule may be engineered by one skilled in the art using conventionally known techniques. In some embodiments, desirable properties may include an ability to bind with low to moderate affinity (e.g., with a K_{D} of about 50 nM or higher, for example, between about 50 nM and about 50 µM, between about 100 nM and about 10 µM, between about 500 nM and about 50 µM) to one or more sites on a molecular barcode. For example, in some aspects, the disclosure provides methods of barcode recognition by detecting reversible binding interactions, and barcode recognition molecules that reversibly bind molecular barcodes with low to moderate affinity advantageously provide more informative binding data and with higher certainty than high affinity binding interactions.

In some embodiments, a barcode recognition molecule binds one or more sites on a molecular barcode with a dissociation constant (K_{D}) of less than about 10⁻⁶ M (e.g., less than about 10⁻⁷ M, less than about 10⁻⁸ M, less than about 10⁻⁹ M, less than about 10⁻¹⁰ M, less than about 10⁻¹¹ M, less than about 10⁻¹² M, to as low as 10⁻¹⁶ M) without significantly binding to other off-target (e.g., non-complementary) sites. In some embodiments, a barcode recognition molecule binds one or more sites on a molecular barcode with a K_{D} of less than about 100 nM, less than about 50 nM, less than about 25 nM, less than about 10 nM, or less than about 1 nM. In some embodiments, a barcode recognition molecule binds one or more sites on a molecular barcode with a K_{D} of between about 50 nM and about 50 µM (e.g., between about 50 nM and about 500 nM, between about 50 nM and about 5 µM, between about 500 nM and about 50 µM, between about 5 µM and about 50 µM, or between about 10 µM and about 50 µM). In some embodiments, a barcode recognition molecule binds one or more sites on a molecular barcode with a K_{D} of about 50 nM.

In some embodiments, a barcode recognition molecule binds one or more sites on a molecular barcode with a dissociation rate (k_{off}) of at least 0.1 s⁻¹. In some embodiments, the dissociation rate is between about 0.1 s⁻¹ and about 1,000 s⁻¹ (e.g., between about 0.5 s⁻¹ and about 500 s⁻¹, between about 0.1 s⁻¹ and about 100 s⁻¹, between about 1 s⁻¹ and about 100 s⁻¹, or between about 0.5 s⁻¹ and about 50 s⁻¹). In some embodiments, the dissociation rate is between about 0.5 s⁻¹ and about 20 s⁻¹. In some embodiments, the dissociation rate is between about 2 s⁻¹ and about 20 s⁻¹. In some embodiments, the dissociation rate is between about 0.5 s⁻¹ and about 2 s⁻¹.

In some embodiments, the value for K_{D} or k_{off} can be a known literature value, or the value can be determined empirically. For example, the value for K_{D} or k_{off} can be measured in a single-molecule assay or an ensemble assay. In some embodiments, the value for k_{off} can be determined empirically based on signal pulse information obtained in a single-molecule assay as described elsewhere herein. For example, the value for k_{off} can be approximated by the reciprocal of the mean pulse duration. In some embodiments, a barcode recognition molecule binds two or more chemically different barcode sites with a different K_{D} or k_{off} for each of the two or more sites. In some embodiments, a first K_{D} or k_{off} for a first site differs from a second K_{D} or k_{off} for a second site by at least 10% (e.g., at least 25%, at least 50%, at least 100%, or more). In some embodiments, the first and second values for K_{D} or k_{off} differ by about 10-25%, 25-50%, 50-75%, 75-100%, or more than 100%, for example by about 2-fold, 3-fold, 4-fold, 5-fold, or more.

As described herein, a barcode recognition molecule may be any biomolecule capable of binding one or more sites on a molecular barcode over other barcode sites. Recognition molecules include, for example, oligonucleotides, nucleic acids, and proteins, any of which may be synthetic or recombinant.

In some embodiments, a barcode recognition molecule is an oligonucleotide (e.g., an oligonucleotide probe). In some embodiments, methods provided herein can be performed by contacting a nucleic acid barcode with an oligonucleotide probe that binds one or more sites on the nucleic acid barcode. In some embodiments, the binding between the oligonucleotide probe and the nucleic acid barcode occurs via hybridization or annealing. Beyond certain experimental conditions (e.g., concentration, temperature), binding properties are in large part driven by length and content of the oligonucleotide probe and its degree of complementarity with the site on the nucleic acid barcode to which it binds (e.g., hybridizes, or anneals). Accordingly, in some embodiments, oligonucleotide probes provide a variety of tunable features for modulating signal pulse characteristics, including, without limitation, length, nucleotide content (e.g., G/C content, nucleotide analogs with different binding characteristics, such as LNA or PNA analogs), degree of complementarity, and experimental factors, such as concentration, temperature, buffer conditions (e.g., pH, salt, magnesium), and DNA denaturing or stabilizing solvents.

In some embodiments, an oligonucleotide probe is at least four nucleotides in length. In some embodiments, an oligonucleotide probe is at least 5, at least 6, at least 7, at least 8, at least 9, at least 10, at least 12, at least 15, at least 20, at least 25, or at least 30 nucleotides in length. In some embodiments, an oligonucleotide probe is fewer than 30 nucleotides in length (e.g., fewer than 25, fewer than 20, fewer than 15, fewer than 12, fewer than 10 nucleotides in length). In some embodiments, an oligonucleotide probe is between about 3 and about 30 nucleotides in length (e.g., between about 3 and about 10, between about 3 and about 8, between about 5 and about 25, between about 5 and about 15, or between about 5 and 10 nucleotides in length). In some embodiments, an oligonucleotide probe is 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleotides in length.

In some embodiments, an oligonucleotide probe can bind to, and provide barcode content information for, one or more barcode sites that are not fully complementary with the oligonucleotide probe. For example, in some embodiments, an oligonucleotide probe binds to one or more barcode sites having a sequence that is less than 100% complementary with the oligonucleotide (e.g., less than 99%, less than 98%, less than 95%, less than 90%, less than 85%, less than 80%, less than 75%, less than 70%, less than 65%, less than 60%, less than 55%, less than 50%, less than 45%, less than 40%, less than 35%, less than 30%, less than 25%, less than 20%, less than 15%, less than 10%, less than 5%, less than 1% or less).

In addition to oligonucleotides, nucleic acid aptamers can be used as barcode recognition molecules in accordance with the disclosure. Nucleic acid aptamers are nucleic acid molecules that have been engineered to bind targets with a desired affinity and selectivity. Accordingly, nucleic acid aptamers may be engineered to bind to a desired barcode site using selection and/or enrichment techniques known in the art. In some embodiments, a barcode recognition molecule comprises a nucleic acid aptamer, such as a DNA aptamer or an RNA aptamer.

In some embodiments, a barcode recognition molecule is a protein or polypeptide. In some embodiments, a recognition molecule is an antibody or an antigen-binding portion of an antibody, an SH2 domain-containing protein or fragment thereof, or an inactivated enzymatic biomolecule, such as a peptidase, an aminotransferase, a ribozyme, an aptazyme, or a tRNA synthetase, including aminoacyl-tRNA synthetases and related molecules described in U.S. Patent Application No. 15/255,433 (Publication No. US2017/0052194A1), filed September 2, 2016, titled "MOLECULES AND METHODS FOR ITERATIVE POLYPEPTIDE ANALYSIS AND PROCESSING."

In some embodiments, a barcode recognition molecule is an amino acid recognition molecule. For example, in some embodiments, a molecular barcode comprises a polypeptide barcode, and an amino acid recognition molecule can be used to decipher barcode content from the polypeptide. In some embodiments, an amino acid recognition molecule binds one or more types of terminal amino acids with different kinetic binding properties. In some embodiments, an amino acid recognition molecule binds different segments of a polypeptide with different kinetic binding properties. For example, in some embodiments, an amino acid recognition molecule binds to polypeptide segments comprising the same type of amino acid at the N-terminus or C-terminus but differing in amino acid content at the penultimate (e.g., n+1) and/or subsequent positions (e.g., different amino acid types at one or more of the second, third, fourth, fifth, or higher, position) relative to the terminal amino acid. These concepts (e.g., differential binding kinetics based on differences in amino acid content only at the penultimate position or higher) and additional examples of amino acid recognition molecules are described more fully in PCT International Publication No. WO2020102741A1, filed November 15, 2019, titled "METHODS AND COMPOSITIONS FOR PROTEIN SEQUENCING".

In some embodiments, methods provided herein comprise contacting a molecular barcode with one or more barcode recognition molecules. For the purposes of this discussion, one or more barcode recognition molecules in the context of a method described herein may be alternatively referred to as a set of barcode recognition molecules. In some embodiments, a set of barcode recognition molecules comprises at least two and up to twenty (e.g., between 2 and 15, between 2 and 10, between 5 and 10, between 10 and 20) barcode recognition molecules. In some embodiments, a set of barcode recognition molecules comprises more than twenty (e.g., 20 to 25, 20 to 30) barcode recognition molecules. It should be appreciated, however, that any number of barcode recognition molecules may be used in accordance with a method of the disclosure to accommodate a desired use.

In accordance with the disclosure, in some embodiments, molecular barcode content can be identified by detecting luminescence from a label attached to a barcode recognition molecule. In some embodiments, a labeled barcode recognition molecule comprises a barcode recognition molecule that binds at least one molecular barcode and a luminescent label having a luminescence that is associated with the barcode recognition molecule. In this way, the luminescence (e.g., luminescence lifetime, luminescence intensity, and other luminescence properties described elsewhere herein, including luminescence-based kinetic binding data) may be associated with the binding of the barcode recognition molecule to identify the at least one molecular barcode. In some embodiments, a plurality of types of labeled barcode recognition molecules may be used in a method according to the disclosure, wherein each type comprises a luminescent label having a luminescence that is uniquely identifiable from among the plurality. Suitable luminescent labels may include luminescent molecules, such as fluorophore dyes, and are described elsewhere herein.

In some embodiments, a barcode recognition molecule comprises a label having binding-induced luminescence. For example, in some embodiments, a labeled aptamer can comprise a donor label and an acceptor label. As a free and unbound molecule, the labeled aptamer adopts a conformation in which the donor and acceptor labels are separated by a distance that limits detectable FRET between the labels (e.g., about 10 nm or more). Upon binding to a barcode site, the labeled aptamer adopts a conformation in which the donor and acceptor labels are within a distance that promotes detectable FRET between the labels (e.g., about 10 nm or less). In yet other embodiments, a labeled aptamer can comprise a quenching moiety and function analogously to a molecular beacon, wherein luminescence is internally quenched as a free molecule and restored upon binding to a barcode site (see, e.g., Hamaguchi, et al. (2001) Analytical Biochemistry 294, 126-131). Similar and alternative labeling strategies would be apparent to those skilled in the art, such as the use of FRET between a labeled aptamer and a labeled molecular barcode. Without wishing to be bound by theory, it is thought that these and other types of mechanisms for binding-induced luminescence may advantageously reduce or eliminate background luminescence to increase overall sensitivity and accuracy of the methods described herein.

In some embodiments, molecular barcode content can be identified by detecting one or more electrical characteristics of a labeled barcode recognition molecule. In some embodiments, a labeled barcode recognition molecule comprises a barcode recognition molecule that binds at least one molecular barcode and a conductivity label that is associated with the barcode recognition molecule. In this way, the one or more electrical characteristics (e.g., charge, current oscillation color, and other electrical characteristics, including conductivity-based kinetic binding data) may be associated with the binding of the barcode recognition molecule to identify the at least one molecular barcode. In some embodiments, a plurality of types of labeled barcode recognition molecules may be used in a method according to the disclosure, wherein each type comprises a conductivity label that produces a change in an electrical signal (e.g., a change in conductance, such as a change in amplitude of conductivity and conductivity transitions of a barcode-specific pattern) that is uniquely identifiable from among the plurality. In some embodiments, the plurality of types of labeled barcode recognition molecules each comprises a conductivity label having a different number of charged groups (e.g., a different number of negatively and/or positively charged groups). Accordingly, in some embodiments, a conductivity label is a charge label. Examples of charge labels include dendrimers, nanoparticles, nucleic acids and other polymers having multiple charged groups. In some embodiments, a conductivity label is uniquely identifiable by its net charge (e.g., a net positive charge or a net negative charge), by its charge density, and/or by its number of charged groups.

### Sequencing

As described herein, in some aspects, the disclosure relates to the discovery of techniques that allow for sequencing and molecular barcode recognition to be performed simultaneously (e.g., in the same reaction mixture), sequentially, and/or on a single surface (e.g., in a single reaction vessel). Accordingly, in some aspects, the disclosure provides methods of analyzing a barcoded biomolecule by: determining the identity of a molecular barcode attached to a biomolecule (e.g., by barcode recognition as described herein); and sequencing the biomolecule.

In some embodiments, the methods comprise: (a) immobilizing a barcoded biomolecule to a surface; (b) determining the identity of the molecular barcode attached to the biomolecule; and (c) sequencing the biomolecule, where (b) and (c) are performed on the surface. In some embodiments, such methods of barcoding and sequencing comprise barcoding and sequencing a plurality of barcoded biomolecules immobilized to a single surface and/or contained within a single reaction vessel (e.g., a sample well). In some embodiments, the methods comprise barcoding and sequencing two or more (e.g., 3 or more, 5 or more, 10 or more, 20 or more, 2-100, 5-50, 5-20, 5-10, 50-100) barcoded biomolecules immobilized to a single surface and/or contained within a single reaction vessel (e.g., a sample well).

**Polypeptide Sequencing.** In some embodiments, the barcoded biomolecule is a polypeptide comprising a molecular barcode. In some embodiments, the identity of the molecular barcode is determined in accordance with methods of barcode recognition described herein. In some embodiments, the methods further comprise sequencing the polypeptide. In some embodiments, polypeptide sequencing is performed by detecting single-molecule binding interactions during a polypeptide degradation process (e.g., as shown in FIG. 2 and described herein).

As used herein, sequencing a polypeptide refers to determining sequence information for a polypeptide. In some embodiments, this can involve determining the identity of each sequential amino acid for a portion (or all) of the polypeptide. However, in some embodiments, this can involve assessing the identity of a subset of amino acids within the polypeptide (e.g., and determining the relative position of one or more amino acid types without determining the identity of each amino acid in the polypeptide). In some embodiments, amino acid content information can be obtained from a polypeptide without directly determining the relative position of different types of amino acids in the polypeptide. The amino acid content alone may be used to infer the identity of the polypeptide that is present (e.g., by comparing the amino acid content to a database of polypeptide information and determining which polypeptide(s) have the same amino acid content).

In some aspects, polypeptide sequencing of a barcoded polypeptide may be performed by identifying one or more types of amino acids of the polypeptide. In some embodiments, one or more amino acids (e.g., terminal amino acids and/or internal amino acids) of the polypeptide are labeled (e.g., directly or indirectly, for example using a binding agent such as an amino acid binding protein), and the relative positions of the labeled amino acids in the polypeptide are determined. In some embodiments, the relative positions of amino acids in a polypeptide are determined using a series of amino acid labeling and cleavage steps.

In some embodiments, the identity of a terminal amino acid (e.g., an N-terminal or a C-terminal amino acid) is assessed after which the terminal amino acid is removed and the identity of the next amino acid at the terminus is assessed, and this process is repeated until a plurality of successive amino acids in the polypeptide are assessed. In some embodiments, assessing the identity of an amino acid comprises determining the type of amino acid that is present. In some embodiments, determining the type of amino acid comprises determining the actual amino acid identity, for example by determining which of the naturally-occurring 20 amino acids is the terminal amino acid (e.g., using a binding agent that is specific for an individual terminal amino acid). In some embodiments, the type of amino acid is selected from alanine, arginine, asparagine, aspartic acid, cysteine, glutamine, glutamic acid, glycine, histidine, isoleucine, leucine, lysine, methionine, phenylalanine, proline, selenocysteine, serine, threonine, tryptophan, tyrosine, and valine. However, in some embodiments assessing the identity of a terminal amino acid type can comprise determining a subset of potential amino acids that can be present at the terminus of the polypeptide. In some embodiments, assessing the identity of a terminal amino acid type comprises determining that an amino acid comprises a post-translational modification.

In some embodiments, a protein or polypeptide can be digested into a plurality of smaller polypeptides and sequence information can be obtained from one or more of these smaller polypeptides (e.g., using a method that involves sequentially assessing a terminal amino acid of a polypeptide and removing that amino acid to expose the next amino acid at the terminus). In some embodiments, methods of polypeptide sequencing may involve subjecting a polypeptide terminus to repeated cycles of terminal amino acid detection and terminal amino acid cleavage.

In some embodiments, polypeptide sequencing comprises providing a polypeptide that is immobilized to a surface of a solid support (e.g., attached to a bottom or sidewall surface of a sample well) through a linkage group. In some embodiments, the linkage group is formed by a covalent or non-covalent linkage between a functionalized terminal end of the polypeptide and a complementary functional moiety attached to the surface. For example, in some embodiments, the linkage group is formed by a non-covalent linkage between a biotin moiety of the polypeptide (e.g., barcoded polypeptide) and an avidin protein of the surface. In some embodiments, the linkage group comprises a nucleic acid.

In some embodiments, the polypeptide is immobilized to the surface through a functionalization moiety at one terminal end such that the other terminal end is free for detecting and cleaving of a terminal amino acid in a sequencing reaction. Accordingly, in some embodiments, the reagents used in certain polypeptide sequencing reactions preferentially interact with terminal amino acids at the non-immobilized (e.g., free) terminus of the polypeptide. In this way, the polypeptide remains immobilized over repeated cycles of detecting and cleaving, e.g., as in a dynamic polypeptide sequencing reaction.

In some embodiments, dynamic polypeptide sequencing is carried out in real-time by evaluating binding interactions of terminal amino acids with labeled amino acid recognition molecules and a cleaving reagent (e.g., an exopeptidase). FIG. 2 shows an example of a method of sequencing in which discrete binding events give rise to signal pulses of a signal output. The inset panel (left) of FIG. 2 illustrates a general scheme of real-time sequencing by this approach. As shown, a labeled amino acid recognition molecule 200 associates with (e.g., binds to) and dissociates from a terminal amino acid (shown here as phenylalanine), which gives rise to a series of pulses in signal output which may be used to identify the terminal amino acid. In some embodiments, the series of pulses provide a pulsing pattern (e.g., a characteristic pattern) which may be diagnostic of the identity of the corresponding terminal amino acid.

As further shown in the inset panel (left) of FIG. 2, in some embodiments, a sequencing reaction mixture further comprises an exopeptidase 201 (e.g., cleaving reagent). In some embodiments, the exopeptidase is present in the mixture at a concentration that is less than that of the labeled amino acid recognition molecule. In some embodiments, the exopeptidase displays broad specificity such that it cleaves most or all types of terminal amino acids. Accordingly, a dynamic sequencing approach can involve monitoring recognition molecule binding at a terminus of a polypeptide over the course of a degradation reaction catalyzed by exopeptidase cleavage activity.

FIG. 2 further shows the progress of signal output intensity over time (right panels). In some embodiments, terminal amino acid cleavage by exopeptidase(s) occurs with lower frequency than the binding pulses of a labeled amino acid recognition molecule. In this way, amino acids of a polypeptide may be counted and/or identified in a real-time sequencing process. In some embodiments, one type of amino acid recognition molecule can associate with more than one type of amino acid, where different characteristic patterns correspond to the association of one type of labeled amino acid recognition molecule with different types of terminal amino acids. For example, in some embodiments, different characteristic patterns (as illustrated by each of phenylalanine (F, Phe), tryptophan (W, Trp), and tyrosine (Y, Tyr)) correspond to the association of one type of labeled amino acid recognition molecule (e.g., ClpS protein) with different types of terminal amino acids over the course of degradation. In some embodiments, a plurality of labeled amino acid recognition molecules may be used, each capable of associating with different subsets of amino acids.

In some embodiments, dynamic peptide sequencing is performed by observing different association events, e.g., association events between an amino acid recognition molecule and an amino acid at a terminal end of a peptide, wherein each association event produces a change in magnitude of a signal, e.g., a luminescence signal, that persists for a duration of time. In some embodiments, observing different association events, e.g., association events between an amino acid recognition molecule and an amino acid at a terminal end of a peptide, can be performed during a peptide degradation process. In some embodiments, a transition from one characteristic signal pattern to another is indicative of amino acid cleavage (e.g., amino acid cleavage resulting from peptide degradation). In some embodiments, amino acid cleavage refers to the removal of at least one amino acid from a terminus of a polypeptide (e.g., the removal of at least one terminal amino acid from the polypeptide). In some embodiments, amino acid cleavage is determined by inference based on a time duration between characteristic signal patterns. In some embodiments, amino acid cleavage is determined by detecting a change in signal produced by association of a labeled cleaving reagent with an amino acid at the terminus of the polypeptide. As amino acids are sequentially cleaved from the terminus of the polypeptide during degradation, a series of changes in magnitude, or a series of signal pulses, is detected.

In some embodiments, signal pulse information may be used to identify an amino acid based on a characteristic pattern in a series of signal pulses. In some embodiments, a characteristic pattern comprises a plurality of signal pulses, each signal pulse comprising a pulse duration. In some embodiments, the plurality of signal pulses may be characterized by a summary statistic (e.g., mean, median, time decay constant) of the distribution of pulse durations in a characteristic pattern. In some embodiments, the mean pulse duration of a characteristic pattern is between about 1 millisecond and about 10 seconds (e.g., between about 1 ms and about 1 s, between about 1 ms and about 100 ms, between about 1 ms and about 10 ms, between about 10 ms and about 10 s, between about 100 ms and about 10 s, between about 1 s and about 10 s, between about 10 ms and about 100 ms, or between about 100 ms and about 500 ms). In some embodiments, different characteristic patterns corresponding to different types of amino acids in a single polypeptide may be distinguished from one another based on a statistically significant difference in the summary statistic. For example, in some embodiments, one characteristic pattern may be distinguishable from another characteristic pattern based on a difference in mean pulse duration of at least 10 milliseconds (e.g., between about 10 ms and about 10 s, between about 10 ms and about 1 s, between about 10 ms and about 100 ms, between about 100 ms and about 10 s, between about 1 s and about 10 s, or between about 100 ms and about 1 s). It should be appreciated that, in some embodiments, smaller differences in mean pulse duration between different characteristic patterns may require a greater number of pulse durations within each characteristic pattern to distinguish one from another with statistical confidence.

Methods and compositions for performing dynamic sequencing are described more fully in PCT International Publication No. WO2020102741A1, filed November 15, 2019, and PCT International Publication No. WO2021236983A2, filed May 20, 2021.

**Nucleic Acid Sequencing.** In some embodiments, the barcoded biomolecule is a nucleic acid comprising a molecular barcode. In some embodiments, an enzyme is bound to the nucleic acid. For example, in some embodiments, the nucleic acid molecule comprises at least one hybridized primer/polymerizing enzyme complex. In some embodiments, the nucleic acid molecule is contacted with a sequencing primer that is complementary to a portion of the nucleic acid molecule such that the sequencing primer anneals to the nucleic acid molecule. This priming location generates a site at which a polymerizing enzyme (e.g., a DNA polymerase) can couple to the nucleic acid molecule to form a hybridized primer/polymerizing enzyme complex. In some embodiments, the identity of the molecular barcode is determined in accordance with methods of barcode recognition described herein. In some embodiments, the methods further comprise sequencing the nucleic acid.

In some embodiments, nucleic acid sequencing is performed by identifying a series of nucleotide monomers that are incorporated into a nascent nucleic acid strand complementary to the nucleic acid of a barcoded biomolecule (e.g., by detecting a time-course of incorporation of a series of labeled nucleotide monomers). In some embodiments, nucleic acid sequencing is performed by identifying a series of nucleotides that are incorporated into a template-dependent nucleic acid sequencing reaction product synthesized by a polymerizing enzyme (e.g., a DNA polymerase).

In some embodiments, methods of nucleic acid sequencing comprise steps of: (i) exposing a complex in a target volume, the complex comprising the barcoded nucleic acid, a primer, and a polymerizing enzyme, to a nucleic acid sequencing reaction composition comprising one or more labeled nucleotides; (ii) directing a series of pulses of one or more excitation energies towards a vicinity of the target volume; (iii) detecting a plurality of emitted photons from labeled nucleotides during sequential incorporation into a nucleic acid strand comprising the primer; and (iv) identifying the sequence of incorporated nucleotides by determining timing, and optionally luminescence intensity, of the emitted photons.

Upon base pairing between a nucleobase of a target nucleic acid (e.g., a barcoded nucleic acid) and the complementary nucleoside polyphosphate (e.g., dNTP), the polymerizing enzyme (e.g., polymerase) incorporates the dNTP into the newly synthesized nucleic acid strand by forming a phosphodiester bond between the 3' hydroxyl end of the newly synthesized strand and the alpha phosphate of the dNTP. In examples in which a label conjugated to the dNTP comprises a fluorophore, its presence is signaled by excitation, and a pulse of emission is detected during and/or after the step of incorporation. For labels that are conjugated to the terminal (gamma) phosphate of the dNTP, incorporation of the dNTP into the newly synthesized strand results in release of the beta and gamma phosphates and the label, which is free to diffuse in the sample well, resulting in a decrease in emission detected from the fluorophore.

In some embodiments, the template-dependent nucleic acid sequencing product is carried out by naturally occurring nucleic acid polymerases. In some embodiments, the polymerase is a mutant or modified variant of a naturally occurring polymerase. In some embodiments, the template-dependent nucleic acid sequence product will comprise one or more nucleotide segments complementary to the template nucleic acid strand. In some embodiments, determining the sequence of a template nucleic acid comprises determining the sequence of its complementary nucleic acid strand.

The term "polymerizing enzyme" or "polymerase," as used herein, generally refers to any enzyme capable of catalyzing a polymerization reaction. Examples of polymerases include, without limitation, a nucleic acid polymerase, a transcriptase or a ligase. Embodiments directed towards single molecule nucleic acid extension (e.g., for nucleic acid sequencing) may use any polymerase that is capable of synthesizing a nucleic acid complementary to a target nucleic acid molecule. In some embodiments, a polymerase may be a DNA polymerase, an RNA polymerase, a reverse transcriptase, and/or a mutant or altered form of one or more thereof.

Examples of polymerases include, but are not limited to, a DNA polymerase, an RNA polymerase, a thermostable polymerase, a wild-type polymerase, a modified polymerase, E. coli DNA polymerase I, T7 DNA polymerase, bacteriophage T4 DNA polymerase φ29 (psi29) DNA polymerase, Taq polymerase, Tth polymerase, Tli polymerase, Pfu polymerase, Pwo polymerase, VENT polymerase, DEEPVENT polymerase, EX-Taq polymerase, LA-Taq polymerase, Sso polymerase, Poc polymerase, Pab polymerase, Mth polymerase, ES4 polymerase, Tru polymerase, Tac polymerase, Tne polymerase, Tma polymerase, Tca polymerase, Tih polymerase, Tfi polymerase, Platinum Taq polymerases, Tbr polymerase, Tfl polymerase, Tth polymerase, Pfutubo polymerase, Pyrobest polymerase, Pwo polymerase, KOD polymerase, Bst polymerase, Sac polymerase, Klenow fragment, polymerase with 3' to 5' exonuclease activity, and variants, modified products and derivatives thereof. In some embodiments, the polymerase is a single subunit polymerase.

During sequencing, a polymerizing enzyme may couple (e.g., attach) to a priming location of a target nucleic acid molecule (e.g., a barcoded nucleic acid). The priming location can be a primer that is complementary to a portion of the target nucleic acid molecule. In some embodiments, the priming location is a gap or nick that is provided within a double stranded segment of the target nucleic acid molecule. A gap or nick can be from 0 to at least 1, 2, 3, 4, 5, 6, 7, 8, 9, 10, 20, 30, or 40 nucleotides in length. A nick can provide a break in one strand of a double stranded sequence, which can provide a priming location for a polymerizing enzyme, such as, for example, a strand displacing polymerase enzyme.

In some cases, a sequencing primer can be annealed to a target nucleic acid molecule (e.g., a barcoded nucleic acid) that may or may not be immobilized to a solid support. A solid support can comprise, for example, a sample well (e.g., a nanoaperture, a reaction chamber) on a chip used for nucleic acid sequencing. In some embodiments, a sequencing primer may be immobilized to a solid support and hybridization of the target nucleic acid molecule also immobilizes the target nucleic acid molecule to the solid support. In some embodiments, a polymerase is immobilized to a solid support and soluble primer and target nucleic acid are contacted to the polymerase. However, in some embodiments a complex comprising a polymerase, a target nucleic acid and a primer is formed in solution and the complex is immobilized to a solid support (e.g., via immobilization of the polymerase, primer, and/or target nucleic acid). In some embodiments, none of the components in a sample well (e.g., a nanoaperture, a reaction chamber) are immobilized to a solid support. For example, in some embodiments, a complex comprising a polymerase, a target nucleic acid, and a primer is formed in solution and the complex is not immobilized to a solid support.

Under appropriate conditions, a polymerase enzyme that is contacted to an annealed primer/target nucleic acid can add or incorporate one or more nucleotides onto the primer, and nucleotides can be added to the primer in a 5' to 3', template-dependent fashion. Such incorporation of nucleotides onto a primer (e.g., via the action of a polymerase) can generally be referred to as a primer extension reaction. Each nucleotide can be associated with a detectable label that can be detected and identified (e.g., based on its luminescent lifetime and/or other characteristics) during the nucleic acid extension reaction and used to determine each nucleotide incorporated into the extended primer and, thus, a sequence of the newly synthesized nucleic acid molecule. Via sequence complementarity of the newly synthesized nucleic acid molecule, the sequence of the target nucleic acid molecule (e.g., a barcoded nucleic acid) can also be determined. In some cases, annealing of a sequencing primer to a target nucleic acid molecule and incorporation of nucleotides to the sequencing primer can occur at similar reaction conditions (e.g., the same or similar reaction temperature) or at differing reaction conditions (e.g., different reaction temperatures). In some embodiments, sequencing by synthesis methods can include the presence of a population of target nucleic acid molecules (e.g., copies of a target nucleic acid) and/or a step of amplification of the target nucleic acid to achieve a population of target nucleic acids. However, in some embodiments, sequencing by synthesis is used to determine the sequence of a single molecule in each reaction that is being evaluated (and nucleic acid amplification is not required to prepare the target template for sequencing). In some embodiments, a plurality of single molecule sequencing reactions are performed in parallel (e.g., on a single chip) according to aspects of the present application. For example, in some embodiments, a plurality of single molecule sequencing reactions are each performed in separate reaction chambers (e.g., nanoapertures, sample wells) on a single chip.

In some embodiments, the target nucleic acid molecule (e.g., a barcoded nucleic acid) used in single molecule sequencing is a single stranded target nucleic acid (e.g., deoxyribonucleic acid (DNA), DNA derivatives, ribonucleic acid (RNA), RNA derivatives) template that is added or immobilized to a sample well (e.g., reaction chamber or reaction vessel) containing at least one additional component of a sequencing reaction (e.g., a polymerase such as, a DNA polymerase, a sequencing primer) immobilized or attached to a solid support such as the bottom or side walls of the sample well. The target nucleic acid molecule or the polymerase can be attached to a sample wall, such as at the bottom or side walls of the sample well directly or through a linker. The sample well also can contain any other reagents needed for nucleic acid synthesis via a primer extension reaction, such as, for example suitable buffers, cofactors, enzymes (e.g., a polymerase) and deoxyribonucleoside polyphosphates, such as deoxyribonucleoside triphosphates, including deoxyadenosine triphosphate (dATP), deoxycytidine triphosphate (dCTP), deoxyguanosine triphosphate (dGTP), deoxyuridine triphosphate (dUTP) and deoxythymidine triphosphate (dTTP) dNTPs, that include luminescent labels.

In some embodiments, each class of dNTPs (e.g., adenine-containing dNTPs (e.g., dATP), cytosine-containing dNTPs (e.g., dCTP), guanine-containing dNTPs (e.g., dGTP), uracil-containing dNTPs (e.g., dUTPs) and thymine-containing dNTPs (e.g., dTTP)) is conjugated to a luminescent molecule that comprises distinct luminescent properties such that detection of light emitted from the luminescent molecule indicates the identity of the dNTP that was incorporated into the newly synthesized nucleic acid. Emitted light from the luminescent molecule (e.g., emitted light from a labeled biomolecule comprising at least one luminescent label) can be detected and attributed to its appropriate luminescent molecule (and, thus, associated dNTP) via any suitable device and/or method. The luminescent molecule may be conjugated to the dNTP at any position such that the presence of the luminescent molecule (e.g., a labeled biomolecule of the application) does not inhibit the incorporation of the dNTP into the newly synthesized nucleic acid strand or the activity of the polymerase. In some embodiments, the luminescent molecule is conjugated to the terminal phosphate (e.g., the gamma phosphate) of the dNTP.

In some embodiments, the single-stranded target nucleic acid template (e.g., a barcoded nucleic acid) can be contacted with a sequencing primer, dNTPs, polymerase and other reagents necessary for nucleic acid synthesis. In some embodiments, all appropriate dNTPs can be contacted with the single-stranded target nucleic acid template simultaneously (e.g., all dNTPs are simultaneously present) such that incorporation of dNTPs can occur continuously. In other embodiments, the dNTPs can be contacted with the single-stranded target nucleic acid template sequentially, where the single-stranded target nucleic acid template is contacted with each appropriate dNTP separately, with washing steps in between contact of the single-stranded target nucleic acid template with differing dNTPs. Such a cycle of contacting the single-stranded target nucleic acid template with each dNTP separately followed by washing can be repeated for each successive base position of the single-stranded target nucleic acid template to be identified.

In some embodiments, the sequencing primer anneals to the single-stranded target nucleic acid template and the polymerase consecutively incorporates the dNTPs (or other nucleoside polyphosphate) to the primer based on the single-stranded target nucleic acid template. The unique luminescent molecule associated with each incorporated dNTP can be excited with the appropriate excitation light during or after incorporation of the dNTP to the primer and its emission can be subsequently detected using any suitable device(s) and/or method(s). Detection of a particular emission of light (e.g., having a particular emission lifetime, intensity, spectrum and/or combination thereof) can be attributed to a particular dNTP incorporated. The sequence obtained from the collection of detected luminescent molecules can then be used to determine the sequence of the single-stranded target nucleic acid template via sequence complementarity.

In some embodiments, the present disclosure provides methods and compositions that may be advantageously utilized in the technologies described in U.S. Patent App. Nos.: 14/543,865, 14/543,867, 14/543,888, 14/821,656, 14/821,686, 14/821,688, 15/161,067, 15/161,088, 15/161,125, 15/255,245, 15/255,303, 15/255,624, 15/261,697, 15/261,724, 15/600,979, 15/846,967, and 15/847,001 (Publication Nos: US2015/0141267A1, US2015/0177150A1, US2015/0141268A1, US2016/0133668A1, US2016/0041095A1, US2016/0084761A1, US2016/0341664A1, US2016/0344156A1, US2017/0107562A1, US2016/0370291A1, US2016/0370292A1, US2016/0369332A1, US2016/0377543A1, US2016/0380025A1, US2017/0362651A1, US2018/0208911A1, and US2018/0223353A1).

### Luminescent Labels

As used herein, a luminescent label is a molecule that absorbs one or more photons and may subsequently emit one or more photons after one or more time durations. In some embodiments, the term is used interchangeably with "label," "detectable label," or "luminescent molecule" depending on context. A luminescent label in accordance with certain embodiments described herein may refer to a luminescent label of a barcode recognition molecule, a luminescent label of a molecular barcode, or a luminescent label of another labeled composition described herein.

In some embodiments, a luminescent label comprises a first and second chromophore. In some embodiments, an excited state of the first chromophore is capable of relaxation via an energy transfer to the second chromophore. In some embodiments, the energy transfer is a Förster resonance energy transfer (FRET). Such a FRET pair may be useful for providing a luminescent label with properties that make the label easier to differentiate from amongst a plurality of luminescent labels in a mixture, or for providing a binding-induced fluorescence that limits background fluorescence as described elsewhere herein. In yet other embodiments, a FRET pair comprises a first chromophore of a first luminescent label and a second chromophore of a second luminescent label-e.g., where FRET occurs between a first label on a barcode recognition molecule and a second label on a molecular barcode. In certain embodiments, the FRET pair may absorb excitation energy in a first spectral range and emit luminescence in a second spectral range.

In some embodiments, a luminescent label refers to a fluorophore or a dye. Typically, a luminescent label comprises an aromatic or heteroaromatic compound and can be a pyrene, anthracene, naphthalene, naphthylamine, acridine, stilbene, indole, benzindole, oxazole, carbazole, thiazole, benzothiazole, benzoxazole, phenanthridine, phenoxazine, porphyrin, quinoline, ethidium, benzamide, cyanine, carbocyanine, salicylate, anthranilate, coumarin, fluoroscein, rhodamine, xanthene, or other like compound.

In some embodiments, a luminescent label comprises a dye selected from one or more of the following: 5/6-Carboxyrhodamine 6G, 5-Carboxyrhodamine 6G, 6-Carboxyrhodamine 6G, 6-TAMRA, Abberior^{®} STAR 440SXP, Abberior^{®} STAR 470SXP, Abberior^{®} STAR 488, Abberior^{®} STAR 512, Abberior^{®} STAR 520SXP, Abberior^{®} STAR 580, Abberior^{®} STAR 600, Abberior^{®} STAR 635, Abberior^{®} STAR 635P, Abberior^{®} STAR RED, Alexa Fluor^{®} 350, Alexa Fluor^{®} 405, Alexa Fluor^{®} 430, Alexa Fluor^{®} 480, Alexa Fluor^{®} 488, Alexa Fluor^{®} 514, Alexa Fluor^{®} 532, Alexa Fluor^{®} 546, Alexa Fluor^{®} 555, Alexa Fluor^{®} 568, Alexa Fluor^{®} 594, Alexa Fluor^{®} 610-X, Alexa Fluor^{®} 633, Alexa Fluor^{®} 647, Alexa Fluor^{®} 660, Alexa Fluor^{®} 680, Alexa Fluor^{®} 700, Alexa Fluor^{®} 750, Alexa Fluor^{®} 790, AMCA, ATTO 390, ATTO 425, ATTO 465, ATTO 488, ATTO 495, ATTO 514, ATTO 520, ATTO 532, ATTO 542, ATTO 550, ATTO 565, ATTO 590, ATTO 610, ATTO 620, ATTO 633, ATTO 647, ATTO 647N, ATTO 655, ATTO 665, ATTO 680, ATTO 700, ATTO 725, ATTO 740, ATTO Oxa12, ATTO Rho101, ATTO Rho11, ATTO Rho12, ATTO Rho13, ATTO Rho14, ATTO Rho3B, ATTO Rho6G, ATTO Thiol2, BD Horizon^{™} V450, BODIPY^{®} 493/501, BODIPY^{®} 530/550, BODIPY^{®} 558/568, BODIPY^{®} 564/570, BODIPY^{®} 576/589, BODIPY^{®} 581/591, BODIPY^{®} 630/650, BODIPY^{®} 650/665, BODIPY^{®} FL, BODIPY^{®} FL-X, BODIPY^{®} R6G, BODIPY^{®} TMR, BODIPY^{®} TR, CAL Fluor^{®} Gold 540, CAL Fluor^{®} Green 510, CAL Fluor^{®} Orange 560, CAL Fluor^{®} Red 590, CAL Fluor^{®} Red 610, CAL Fluor^{®} Red 615, CAL Fluor^{®} Red 635, Cascade^{®} Blue, CF^{™}350, CF^{™}405M, CF^{™}405S, CF^{™}488_{A}, CF^{™}514, CF^{™}532, CF^{™}543, CF^{™}546, CF^{™}S555, CF^{™}S568, CF^{™}594, CF^{™}620R, CF^{™}633, CF^{™}633-V1, CF^{™}640R, CF^{™}640R-V1, CF^{™}640R-V2\, CF^{™}660C, CF^{™}660R, CF^{™}680, CF^{™}680R, CF^{™}680R-V1, CF^{™}750, CF^{™}770, CF^{™}790, Chromeo^{™} 642, Chromis 425N, Chromis 500N, Chromis 515N, Chromis 530N, Chromis 550A, Chromis 550C, Chromis 550Z, Chromis 560N, Chromis 570N, Chromis 577N, Chromis 600N, Chromis 630N, Chromis 645A, Chromis 645C, Chromis 645Z, Chromis 678A, Chromis 678C, Chromis 678Z, Chromis 770A, Chromis 770C, Chromis 800A, Chromis 800C, Chromis 830A, Chromis 830C, Cy^{®}3, Cy^{®}3.5, Cy^{®}3B, Cy^{®}5, Cy^{®}5.5, Cy^{®}7, DyLight^{®} 350, DyLight^{®} 405, DyLight^{®} 415-Col, DyLight^{®} 425Q, DyLight^{®} 485-LS, DyLight^{®} 488, DyLight^{®} 504Q, DyLight^{®} 510-LS, DyLight^{®} 515-LS, DyLight^{®} 521-LS, DyLight^{®} 530-R2, DyLight^{®} 543Q, DyLight^{®} 550, DyLight^{®} 554-R0, DyLight^{®} 554-R1, DyLight^{®} 590-R2, DyLight^{®} 594, DyLight^{®} 610-B1, DyLight^{®} 615-B2, DyLight^{®} 633, DyLight^{®} 633-B1, DyLight^{®} 633-B2, DyLight^{®} 650, DyLight^{®} 655-B1, DyLight^{®} 655-B2, DyLight^{®} 655-B3, DyLight^{®} 655-B4, DyLight^{®} 662Q, DyLight^{®} 675-B1, DyLight^{®} 675-B2, DyLight^{®} 675-B3, DyLight^{®} 675-B4, DyLight^{®} 679-C5, DyLight^{®} 680, DyLight^{®} 683Q, DyLight^{®} 690-B1, DyLight^{®} 690-B2, DyLight^{®} 696Q, DyLight^{®} 700-B1, DyLight^{®} 700-B1, DyLight^{®} 730-B1, DyLight^{®} 730-B2, DyLight^{®} 730-B3, DyLight^{®} 730-B4, DyLight^{®} 747, DyLight^{®} 747-B1, DyLight^{®} 747-B2, DyLight^{®} 747-B3, DyLight^{®} 747-B4, DyLight^{®} 755, DyLight^{®} 766Q, DyLight^{®} 775-B2, DyLight^{®} 775-B3, DyLight^{®} 775-B4, DyLight^{®} 780-B1, DyLight^{®} 780-B2, DyLight^{®} 780-B3, DyLight^{®} 800, DyLight^{®} 830-B2, Dyomics-350, Dyomics-350XL, Dyomics-360XL, Dyomics-370XL, Dyomics-375XL, Dyomics-380XL, Dyomics-390XL, Dyomics-405, Dyomics-415, Dyomics-430, Dyomics-431, Dyomics-478, Dyomics-480XL, Dyomics-481XL, Dyomics-485XL, Dyomics-490, Dyomics-495, Dyomics-505, Dyomics-510XL, Dyomics-511XL, Dyomics-520XL, Dyomics-521XL, Dyomics-530, Dyomics-547, Dyomics-547P1, Dyomics-548, Dyomics-549, Dyomics-549P1, Dyomics-550, Dyomics-554, Dyomics-555, Dyomics-556, Dyomics-560, Dyomics-590, Dyomics-591, Dyomics-594, Dyomics-601XL, Dyomics-605, Dyomics-610, Dyomics-615, Dyomics-630, Dyomics-631, Dyomics-632, Dyomics-633, Dyomics-634, Dyomics-635, Dyomics-636, Dyomics-647, Dyomics-647P1, Dyomics-648, Dyomics-648P1, Dyomics-649, Dyomics-649P1, Dyomics-650, Dyomics-651, Dyomics-652, Dyomics-654, Dyomics-675, Dyomics-676, Dyomics-677, Dyomics-678, Dyomics-679P1, Dyomics-680, Dyomics-681, Dyomics-682, Dyomics-700, Dyomics-701, Dyomics-703, Dyomics-704, Dyomics-730, Dyomics-731, Dyomics-732, Dyomics-734, Dyomics-749, Dyomics-749P1, Dyomics-750, Dyomics-751, Dyomics-752, Dyomics-754, Dyomics-776, Dyomics-777, Dyomics-778, Dyomics-780, Dyomics-781, Dyomics-782, Dyomics-800, Dyomics-831, eFluor^{®} 450, Eosin, FITC, Fluorescein, HiLyte^{™} Fluor 405, HiLyte^{™} Fluor 488, HiLyte^{™} Fluor 532, HiLyte^{™} Fluor 555, HiLyte^{™} Fluor 594, HiLyte^{™} Fluor 647, HiLyte^{™} Fluor 680, HiLyte^{™} Fluor 750, IRDye^{®} 680LT, IRDye^{®} 750, IRDye^{®} 800CW, JOE, LightCycler^{®} 640R, LightCycler^{®} Red 610, LightCycler^{®} Red 640, LightCycler^{®} Red 670, LightCycler^{®} Red 705, Lissamine Rhodamine B, Napthofluorescein, Oregon Green^{®} 488, Oregon Green^{®} 514, Pacific Blue^{™}, Pacific Green^{™}, Pacific Orange^{™}, PET, PF350, PF405, PF415, PF488, PF505, PF532, PF546, PF555P, PF568, PF594, PF610, PF633P, PF647P, Quasar^{®} 570, Quasar^{®} 670, Quasar^{®} 705, Rhodamine 123, Rhodamine 6G, Rhodamine B, Rhodamine Green, Rhodamine Green-X, Rhodamine Red, ROX, Seta^{™} 375, Seta^{™} 470, Seta^{™} 555, Seta^{™} 632, Seta^{™} 633, Seta^{™} 650, Seta^{™} 660, Seta^{™} 670, Seta^{™} 680, Seta^{™} 700, Seta^{™} 750, Seta^{™} 780, Seta^{™} APC-780, Seta^{™} PerCP-680, Seta^{™} R-PE-670, Seta^{™} 646, SeTau 380, SeTau 425, SeTau 647, SeTau 405, Square 635, Square 650, Square 660, Square 672, Square 680, Sulforhodamine 101, TAMRA, TET, Texas Red^{®}, TMR, TRITC, Yakima Yellow^{™}, Zenon^{®}, Zy3, Zy5, Zy5.5, and Zy7.

### Luminescence

In some aspects, the disclosure relates to barcode recognition based on one or more luminescence properties of a luminescent label. In some embodiments, a luminescent label is identified based on luminescence lifetime, luminescence intensity, brightness, absorption spectra, emission spectra, luminescence quantum yield, or a combination of two or more thereof. In some embodiments, a plurality of types of luminescent labels can be distinguished from each other based on different luminescence lifetimes, luminescence intensities, brightnesses, absorption spectra, emission spectra, luminescence quantum yields, or combinations of two or more thereof.

In some embodiments, luminescence is detected by exposing a luminescent label to a series of separate light pulses and evaluating the timing or other properties of each photon that is emitted from the label. In some embodiments, information for a plurality of photons emitted sequentially from a label is aggregated and evaluated to identify the label and thereby identify an associated barcode site. In some embodiments, a luminescence lifetime of a label is determined from a plurality of photons that are emitted sequentially from the label, and the luminescence lifetime can be used to identify the label. In some embodiments, a luminescence intensity of a label is determined from a plurality of photons that are emitted sequentially from the label, and the luminescence intensity can be used to identify the label. In some embodiments, a luminescence lifetime and luminescence intensity of a label is determined from a plurality of photons that are emitted sequentially from the label, and the luminescence lifetime and luminescence intensity can be used to identify the label.

In some aspects of the disclosure, a single molecule is exposed to a plurality of separate light pulses and a series of emitted photons are detected and analyzed. In some embodiments, the series of emitted photons provides information about the single molecule that is present and that does not change in the mixture over the course of an experiment. However, in some embodiments, the series of emitted photons provides information about a series of different molecules that are present at different times in the mixture (e.g., as a reaction or process progresses).

In certain embodiments, a luminescent label absorbs one photon and emits one photon after a time duration. In some embodiments, the luminescence lifetime of a label can be determined or estimated by measuring the time duration. In some embodiments, the luminescence lifetime of a label can be determined or estimated by measuring a plurality of time durations for multiple pulse events and emission events. In some embodiments, the luminescence lifetime of a label can be differentiated amongst the luminescence lifetimes of a plurality of types of labels by measuring the time duration. In some embodiments, the luminescence lifetime of a label can be differentiated amongst the luminescence lifetimes of a plurality of types of labels by measuring a plurality of time durations for multiple pulse events and emission events. In certain embodiments, a label is identified or differentiated amongst a plurality of types of labels by determining or estimating the luminescence lifetime of the label. In certain embodiments, a label is identified or differentiated amongst a plurality of types of labels by differentiating the luminescence lifetime of the label amongst a plurality of the luminescence lifetimes of a plurality of types of labels.

Determination of a luminescence lifetime of a luminescent label can be performed using any suitable method (e.g., by measuring the lifetime using a suitable technique or by determining time-dependent characteristics of emission). In some embodiments, determining the luminescence lifetime of one label comprises determining the lifetime relative to another label. In some embodiments, determining the luminescence lifetime of a label comprises determining the lifetime relative to a reference. In some embodiments, determining the luminescence lifetime of a label comprises measuring the lifetime (e.g., fluorescence lifetime). In some embodiments, determining the luminescence lifetime of a label comprises determining one or more temporal characteristics that are indicative of lifetime. In some embodiments, the luminescence lifetime of a label can be determined based on a distribution of a plurality of emission events (e.g., 1, 2, 3, 4, 5, 6, 7, 8, 9, 10, 11, 12, 13, 14, 15, 16, 17, 18, 19, 20, 30, 40, 50, 60, 70, 80, 90, 100, or more emission events) occurring across one or more time-gated windows relative to an excitation pulse. For example, a luminescence lifetime of a label can be distinguished from a plurality of labels having different luminescence lifetimes based on the distribution of photon arrival times measured with respect to an excitation pulse.

It should be appreciated that a luminescence lifetime of a luminescent label is indicative of the timing of photons emitted after the label reaches an excited state and the label can be distinguished by information indicative of the timing of the photons. Some embodiments may include distinguishing a label from a plurality of labels based on the luminescence lifetime of the label by measuring times associated with photons emitted by the label. The distribution of times may provide an indication of the luminescence lifetime which may be determined from the distribution. In some embodiments, the label is distinguishable from the plurality of labels based on the distribution of times, such as by comparing the distribution of times to a reference distribution corresponding to a known label. In some embodiments, a value for the luminescence lifetime is determined from the distribution of times.

As used herein, in some embodiments, luminescence intensity refers to the number of emitted photons per unit time that are emitted by a luminescent label which is being excited by delivery of a pulsed excitation energy. In some embodiments, the luminescence intensity refers to the detected number of emitted photons per unit time that are emitted by a label which is being excited by delivery of a pulsed excitation energy, and are detected by a particular sensor or set of sensors.

As used herein, in some embodiments, brightness refers to a parameter that reports on the average emission intensity per luminescent label. Thus, in some embodiments, "emission intensity" may be used to generally refer to brightness of a composition comprising one or more labels. In some embodiments, brightness of a label is equal to the product of its quantum yield and extinction coefficient.

As used herein, in some embodiments, luminescence quantum yield refers to the fraction of excitation events at a given wavelength or within a given spectral range that lead to an emission event, and is typically less than 1. In some embodiments, the luminescence quantum yield of a luminescent label described herein is between 0 and about 0.001, between about 0.001 and about 0.01, between about 0.01 and about 0.1, between about 0.1 and about 0.5, between about 0.5 and 0.9, or between about 0.9 and 1. In some embodiments, a label is identified by determining or estimating the luminescence quantum yield.

As used herein, in some embodiments, an excitation energy is a pulse of light from a light source. In some embodiments, an excitation energy is in the visible spectrum. In some embodiments, an excitation energy is in the ultraviolet spectrum. In some embodiments, an excitation energy is in the infrared spectrum. In some embodiments, an excitation energy is at or near the absorption maximum of a luminescent label from which a plurality of emitted photons are to be detected. In certain embodiments, the excitation energy is between about 500 nm and about 700 nm (e.g., between about 500 nm and about 600 nm, between about 600 nm and about 700 nm, between about 500 nm and about 550 nm, between about 550 nm and about 600 nm, between about 600 nm and about 650 nm, or between about 650 nm and about 700 nm). In certain embodiments, an excitation energy may be monochromatic or confined to a spectral range. In some embodiments, a spectral range has a range of between about 0.1 nm and about 1 nm, between about 1 nm and about 2 nm, or between about 2 nm and about 5 nm. In some embodiments, a spectral range has a range of between about 5 nm and about 10 nm, between about 10 nm and about 50 nm, or between about 50 nm and about 100 nm.

### Devices and Systems

Methods in accordance with the disclosure, in some aspects, may involve immobilizing a molecular barcode on a surface of a substrate. In some embodiments, the substrate is a solid support, such as a biosensor, a microarray, a chip, or an integrated device as described herein. In some embodiments, a plurality of molecular barcodes are attached to a plurality of sites (e.g., with each site having one molecular barcode of the plurality attached thereto) on an array. In some embodiments, a molecular barcode may be immobilized on a surface of a sample well (e.g., on a bottom surface of the sample well) on a substrate comprising an array of sample wells. In some embodiments, a molecular barcode is immobilized (e.g., attached to the surface) directly or indirectly (e.g., through a linker or through an analyte that is attached to the surface). The immobilized molecular barcode can be attached using any suitable covalent or non-covalent linker or linkage group, for example, as described in this disclosure.

In some embodiments, a molecular barcode is attached to a surface through a covalent linkage group, which may be formed using techniques (e.g., click chemistry) known in the art. In some embodiments, a molecular barcode is attached to a surface through a non-covalent linkage group. In some embodiments, the non-covalent linkage group comprises an avidin protein. Avidin proteins are biotin-binding proteins, generally having a biotin binding site at each of four subunits of the avidin protein. Avidin proteins include, for example, avidin, streptavidin, traptavidin, tamavidin, bradavidin, xenavidin, and homologs and variants thereof. In some cases, the monomeric, dimeric, or tetrameric form of the avidin protein can be used. In some embodiments, the avidin protein of an avidin protein complex is streptavidin in a tetrameric form (e.g., a homotetramer). In some embodiments, the biotin binding sites of an avidin protein provide attachment points for a biotinylated surface, a biotinylated molecular barcode, and/or a biotinylated analyte.

The multivalency of avidin proteins can allow for various linkage configurations. For example, in some embodiments, a biotin linkage moiety can be used to provide a single point of attachment to an avidin protein. In some embodiments, a bis-biotin linkage moiety can be used to provide two points of attachment to an avidin protein. In some embodiments, a barcode construct of the disclosure is immobilized to a surface through an avidin protein complex formed by two bis-biotin linkage moieties. In some embodiments, the barcode construct comprises one of the two bis-biotin linkage moieties, and the surface comprises the other of the two bis-biotin linkage moieties. Further examples of suitable compositions and methods for single-molecule surface immobilization are described in U.S. Patent Application No. 17/067,184 (Publication No. US2021/0129179A1), filed October 9, 2020, titled "SURFACE MODIFICATION IN THE VAPOR PHASE," and U.S. Patent Application No. 15/971,493 (Publication No. US2018/0326412A1), filed May 4, 2018, titled "SUBSTRATES HAVING MODIFIED SURFACE REACTIVITY AND ANTIFOULING PROPERTIES IN BIOLOGICAL REACTIONS".

In some aspects, the disclosure provides an apparatus comprising a substrate having an array of single-molecule confinement sites. In some embodiments, a plurality of the single-molecule confinement sites each comprise a single molecule comprising a molecular barcode as described herein. In some embodiments, the molecular barcode is immobilized to a surface of the single-molecule confinement site. In some embodiments, the apparatus comprises a receptacle or other means for keeping reagents (e.g., one or more barcode recognition molecules, or any one or more of the compositions described herein) in contact with the substrate. Accordingly, in some embodiments, the substrate comprises a plurality of different molecular barcodes in contact with one or more barcode recognition molecules of the disclosure. In some embodiments, the plurality of different molecular barcodes and the one or more barcode recognition molecules interact in accordance with the binding parameters (e.g., K_{D}, k_{off}, kₒₙ, pulse duration, interpulse duration, and other signal characteristics) as described elsewhere herein. In some embodiments, the substrate is an integrated device. In some embodiments, the plurality of the single-molecule confinement sites comprise a plurality of sample wells.

Methods in accordance with the disclosure, in some aspects, may be performed using a system that permits single-molecule analysis. The system may include an integrated device and an instrument configured to interface with the integrated device. The integrated device may include an array of pixels, where individual pixels include a sample well and at least one photodetector. The sample wells of the integrated device may be formed on or through a surface of the integrated device and be configured to receive a sample placed on the surface of the integrated device. Collectively, the sample wells may be considered as an array of sample wells. The plurality of sample wells may have a suitable size and shape such that at least a portion of the sample wells receive a single sample (e.g., a single molecule, such as a polypeptide). In some embodiments, the number of samples within a sample well may be distributed among the sample wells of the integrated device such that some sample wells contain one sample while others contain zero, two or more samples.

Excitation light is provided to the integrated device from one or more light source external to the integrated device. Optical components of the integrated device may receive the excitation light from the light source and direct the light towards the array of sample wells of the integrated device and illuminate an illumination region within the sample well. In some embodiments, a sample well may have a configuration that allows for the sample to be retained in proximity to a surface of the sample well, which may ease delivery of excitation light to the sample and detection of emission light from the sample. A sample positioned within the illumination region may emit emission light in response to being illuminated by the excitation light. For example, the sample may be labeled with a fluorescent label, which emits light in response to achieving an excited state through the illumination of excitation light. Emission light emitted by a sample may then be detected by one or more photodetectors within a pixel corresponding to the sample well with the sample being analyzed. When performed across the array of sample wells, which may range in number between approximately 10,000 pixels to 1,000,000 pixels according to some embodiments, multiple samples can be analyzed in parallel.

The integrated device may include an optical system for receiving excitation light and directing the excitation light among the sample well array. The optical system may include one or more grating couplers configured to couple excitation light to other optical components of the integrated device and direct the excitation light to the other optical components. For example, the optical system may include optical components that direct the excitation light from the grating coupler(s) towards the sample well array. Such optical components may include optical splitters, optical combiners, and waveguides. In some embodiments, one or more optical splitters may couple excitation light from a grating coupler and deliver excitation light to at least one of the waveguides. According to some embodiments, the optical splitter may have a configuration that allows for delivery of excitation light to be substantially uniform across all the waveguides such that each of the waveguides receives a substantially similar amount of excitation light. Such embodiments may improve performance of the integrated device by improving the uniformity of excitation light received by sample wells of the integrated device. Examples of suitable components, e.g., for coupling excitation light to a sample well and/or directing emission light to a photodetector, to include in an integrated device are described in U.S. Patent Application No. 14/821,688 (Publication No. US2016/0084761A1), filed August 7, 2015, titled "INTEGRATED DEVICE FOR PROBING, DETECTING AND ANALYZING MOLECULES," and U.S. Patent Application No. 14/543,865 (Publication No. US2015/0141267A1), filed November 17, 2014, titled "INTEGRATED DEVICE WITH EXTERNAL LIGHT SOURCE FOR PROBING, DETECTING, AND ANALYZING MOLECULES". Examples of suitable grating couplers and waveguides that may be implemented in the integrated device are described in U.S. Patent Application No. 15/844,403 (Publication No. US2018/0172906A1), filed December 15, 2017, titled "OPTICAL COUPLER AND WAVEGUIDE SYSTEM".

Additional photonic structures may be positioned between the sample wells and the photodetectors and configured to reduce or prevent excitation light from reaching the photodetectors, which may otherwise contribute to signal noise in detecting emission light. In some embodiments, metal layers which may act as a circuitry for the integrated device, may also act as a spatial filter. Examples of suitable photonic structures may include spectral filters, a polarization filters, and spatial filters and are described in U.S. Patent Application No. 16/042,968 (Publication No. US2019/0025511A1), filed July 23, 2018, titled "OPTICAL REJECTION PHOTONIC STRUCTURES," and U.S. Provisional Patent Application No. 63/124,655, filed December 11, 2020, titled "INTEGRATED CIRCUIT WITH IMPROVED CHARGE TRANSFER EFFICIENCY AND ASSOCIATED TECHNIQUES".

Components located off the integrated device may be used to position and align an excitation source to the integrated device. Such components may include optical components including lenses, mirrors, prisms, windows, apertures, attenuators, and/or optical fibers. Additional mechanical components may be included in the instrument to allow for control of one or more alignment components. Such mechanical components may include actuators, stepper motors, and/or knobs. Examples of suitable excitation sources and alignment mechanisms are described in U.S. Patent Application No. 15/161,088 (Publication No. US2016/0344156A1), filed May 20, 2016, titled "PULSED LASER AND SYSTEM". Another example of a beam-steering module is described in U.S. Patent Application No. 15/842,720 (Publication No. US2018/0173000A1), filed December 14, 2017, titled "COMPACT BEAM SHAPING AND STEERING ASSEMBLY". Additional examples of suitable excitation sources are described in U.S. Patent Application No. 14/821,688 (Publication No. US2016/0084761A1), filed August 7, 2015, titled "INTEGRATED DEVICE FOR PROBING, DETECTING AND ANALYZING MOLECULES".

The photodetector(s) positioned with individual pixels of the integrated device may be configured and positioned to detect emission light from the pixel's corresponding sample well. Examples of suitable photodetectors are described in U.S. Patent Application No. 14/821,656 (Publication No. US2016/0133668A1), filed August 7, 2015, titled "INTEGRATED DEVICE FOR TEMPORAL BINNING OF RECEIVED PHOTONS". In some embodiments, a sample well and its respective photodetector(s) may be aligned along a common axis. In this manner, the photodetector(s) may overlap with the sample well within the pixel.

Characteristics of the detected emission light may provide an indication for identifying the label associated with the emission light. Such characteristics may include any suitable type of characteristic, including an arrival time of photons detected by a photodetector, an amount of photons accumulated over time by a photodetector, and/or a distribution of photons across two or more photodetectors. In some embodiments, such characteristics can be any one or a combination of two or more of luminescence lifetime, luminescence intensity, brightness, absorption spectra, emission spectra, luminescence quantum yield, wavelength (e.g., peak wavelength), and signal characteristics (e.g., pulse duration, interpulse durations, change in signal magnitude).

In some embodiments, a photodetector may have a configuration that allows for the detection of one or more timing characteristics associated with a sample's emission light (e.g., luminescence lifetime). The photodetector may detect a distribution of photon arrival times after a pulse of excitation light propagates through the integrated device, and the distribution of arrival times may provide an indication of a timing characteristic of the sample's emission light (e.g., a proxy for luminescence lifetime). In some embodiments, the one or more photodetectors provide an indication of the probability of emission light emitted by the label (e.g., luminescence intensity). In some embodiments, a plurality of photodetectors may be sized and arranged to capture a spatial distribution of the emission light. Output signals from the one or more photodetectors may then be used to distinguish a label from among a plurality of labels, where the plurality of labels may be used to identify a sample within the sample. In some embodiments, a sample may be excited by multiple excitation energies, and emission light and/or timing characteristics of the emission light emitted by the sample in response to the multiple excitation energies may distinguish a label from a plurality of labels.

In operation, parallel analyses of samples within the sample wells are carried out by exciting some or all of the samples within the wells using excitation light and detecting signals from sample emission with the photodetectors. Emission light from a sample may be detected by a corresponding photodetector and converted to at least one electrical signal. The electrical signals may be transmitted along conducting lines in the circuitry of the integrated device, which may be connected to an instrument interfaced with the integrated device. The electrical signals may be subsequently processed and/or analyzed. Processing or analyzing of electrical signals may occur on a suitable computing device either located on or off the instrument.

The instrument may include a user interface for controlling operation of the instrument and/or the integrated device. The user interface may be configured to allow a user to input information into the instrument, such as commands and/or settings used to control the functioning of the instrument. In some embodiments, the user interface may include buttons, switches, dials, and a microphone for voice commands. The user interface may allow a user to receive feedback on the performance of the instrument and/or integrated device, such as proper alignment and/or information obtained by readout signals from the photodetectors on the integrated device. In some embodiments, the user interface may provide feedback using a speaker to provide audible feedback. In some embodiments, the user interface may include indicator lights and/or a display screen for providing visual feedback to a user.

In some embodiments, the instrument may include a computer interface configured to connect with a computing device. The computer interface may be a USB interface, a FireWire interface, or any other suitable computer interface. A computing device may be any general purpose computer, such as a laptop or desktop computer. In some embodiments, a computing device may be a server (e.g., cloud-based server) accessible over a wireless network via a suitable computer interface. The computer interface may facilitate communication of information between the instrument and the computing device. Input information for controlling and/or configuring the instrument may be provided to the computing device and transmitted to the instrument via the computer interface. Output information generated by the instrument may be received by the computing device via the computer interface. Output information may include feedback about performance of the instrument, performance of the integrated device, and/or data generated from the readout signals of the photodetector.

In some embodiments, the instrument may include a processing device configured to analyze data received from one or more photodetectors of the integrated device and/or transmit control signals to the excitation source(s). In some embodiments, the processing device may comprise a general purpose processor, a specially-adapted processor (e.g., a central processing unit (CPU) such as one or more microprocessor or microcontroller cores, a field-programmable gate array (FPGA), an application-specific integrated circuit (ASIC), a custom integrated circuit, a digital signal processor (DSP), or a combination thereof). In some embodiments, the processing of data from one or more photodetectors may be performed by both a processing device of the instrument and an external computing device. In other embodiments, an external computing device may be omitted and processing of data from one or more photodetectors may be performed solely by a processing device of the integrated device.

According to some embodiments, the instrument that is configured to analyze samples based on luminescence emission characteristics may detect differences in luminescence lifetimes and/or intensities between different luminescent molecules, and/or differences between lifetimes and/or intensities of the same luminescent molecules in different environments. The inventors have recognized and appreciated that differences in luminescence emission lifetimes can be used to discern between the presence or absence of different luminescent molecules and/or to discern between different environments or conditions to which a luminescent molecule is subjected. In some cases, discerning luminescent molecules based on lifetime (rather than emission wavelength, for example) can simplify aspects of the system. As an example, wavelength-discriminating optics (such as wavelength filters, dedicated detectors for each wavelength, dedicated pulsed optical sources at different wavelengths, and/or diffractive optics) may be reduced in number or eliminated when discerning luminescent molecules based on lifetime. In some cases, a single pulsed optical source operating at a single characteristic wavelength may be used to excite different luminescent molecules that emit within a same wavelength region of the optical spectrum but have measurably different lifetimes. An analytic system that uses a single pulsed optical source, rather than multiple sources operating at different wavelengths, to excite and discern different luminescent molecules emitting in a same wavelength region can be less complex to operate and maintain, more compact, and may be manufactured at lower cost.

Although analytic systems based on luminescence lifetime analysis may have certain benefits, the amount of information obtained by an analytic system and/or detection accuracy may be increased by allowing for additional detection techniques. For example, some embodiments of the systems may additionally be configured to discern one or more properties of a sample based on luminescence wavelength and/or luminescence intensity. In some implementations, luminescence intensity may be used additionally or alternatively to distinguish between different luminescent labels. For example, some luminescent labels may emit at significantly different intensities or have a significant difference in their probabilities of excitation (e.g., at least a difference of about 35%) even though their decay rates may be similar. By referencing binned signals to measured excitation light, it may be possible to distinguish different luminescent labels based on intensity levels.

According to some embodiments, different luminescence lifetimes may be distinguished with a photodetector that is configured to time-bin luminescence emission events following excitation of a luminescent label. The time binning may occur during a single charge-accumulation cycle for the photodetector. A charge-accumulation cycle is an interval between read-out events during which photo-generated carriers are accumulated in bins of the time-binning photodetector. Examples of a time-binning photodetector are described in U.S. Patent Application No. 14/821,656 (Publication No. US2016/0133668A1), filed August 7, 2015, titled "INTEGRATED DEVICE FOR TEMPORAL BINNING OF RECEIVED PHOTONS". In some embodiments, a time-binning photodetector may generate charge carriers in a photon absorption/carrier generation region and directly transfer charge carriers to a charge carrier storage bin in a charge carrier storage region. In such embodiments, the time-binning photodetector may not include a carrier travel/capture region. Such a time-binning photodetector may be referred to as a "direct binning pixel." Examples of time-binning photodetectors, including direct binning pixels, are described in U.S. Patent Application No. 15/852,571 (Publication No. US2018/0180546A1), filed December 22, 2017, titled "INTEGRATED PHOTODETECTOR WITH DIRECT BINNING PIXEL".

In some embodiments, different numbers of fluorophores of the same type may be linked to different reagents in a sample, so that each reagent may be identified based on luminescence intensity. For example, two fluorophores may be linked to a first labeled recognition molecule and four or more fluorophores may be linked to a second labeled recognition molecule. Because of the different numbers of fluorophores, there may be different excitation and fluorophore emission probabilities associated with the different recognition molecules. For example, there may be more emission events for the second labeled recognition molecule during a signal accumulation interval, so that the apparent intensity of the bins is significantly higher than for the first labeled recognition molecule.

The inventors have recognized and appreciated that distinguishing biological or chemical samples based on fluorophore decay rates and/or fluorophore intensities may enable a simplification of the optical excitation and detection systems. For example, optical excitation may be performed with a single-wavelength source (e.g., a source producing one characteristic wavelength rather than multiple sources or a source operating at multiple different characteristic wavelengths). Additionally, wavelength discriminating optics and filters may not be needed in the detection system. Also, a single photodetector may be used for each sample well to detect emission from different fluorophores. The phrase "characteristic wavelength" or "wavelength" is used to refer to a central or predominant wavelength within a limited bandwidth of radiation (e.g., a central or peak wavelength within a 20 nm bandwidth output by a pulsed optical source). In some cases, "characteristic wavelength" or "wavelength" may be used to refer to a peak wavelength within a total bandwidth of radiation output by a source.

### Exemplary Integrated Device

According to an aspect of the present disclosure, an exemplary integrated device may be configured to perform single-molecule analysis in combination with an instrument as described above. It should be appreciated that the exemplary integrated device described herein is intended to be illustrative and that other integrated device configurations may be configured to perform any or all techniques described herein.

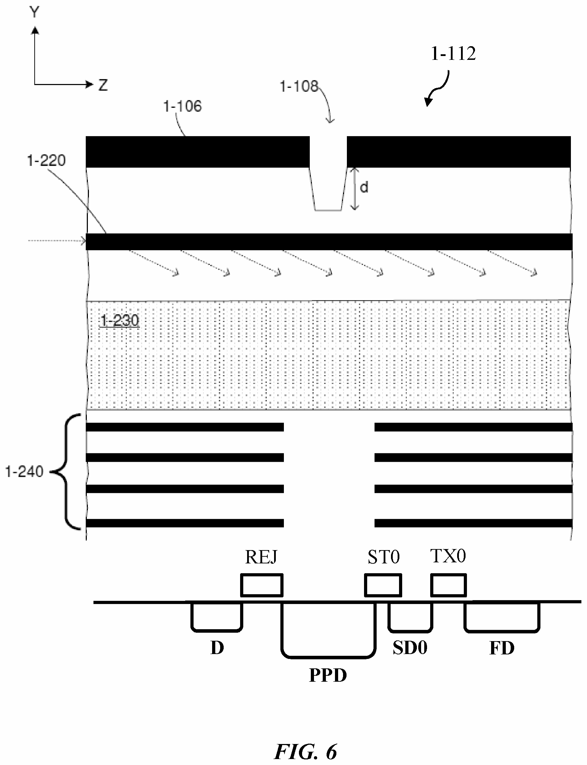

FIG. 6 illustrates a cross-sectional view of a pixel 1-112 of an integrated device 1-102. Pixel 1-112 includes a photodetection region, which may be a pinned photodiode (PPD), and a charge storage region, which may be a storage diode (SD0). In some embodiments, a photodetection region and charge storage regions may be formed in semiconductor material of a pixel by doping regions of the semiconductor material. For example, the photodetection region and charge storage regions can be formed using a same conductivity type (e.g., n-type doping or p-type doping).

During operation of pixel 1-112, excitation light may illuminate sample well 1-108 causing incident photons, including fluorescence emissions from a sample, to flow along the optical axis to photodetection region PPD. As shown in FIG. 6, pixel 1-112 may include a waveguide 1-220 configured to optically (e.g., evanescently) couple excitation light from a grating coupler of the integrated device (not shown) to the sample well 1-108. In response, a sample in the sample well 1-108 may emit fluorescent light toward photodetection region PPD. In some embodiments, pixel 1-112 may also include one or more photonic structures 1-230, which may include one or more optical rejection structures such as a spectral filter, a polarization filter, and/or a spatial filter. For example, the photonic structures 1-230 may be configured to reduce the amount of excitation light that reaches the photodetection region PPD and/or increase the amount of fluorescent emissions that reach the photodetection region PPD. Also shown in pixel 1-112, pixel 1-112 may include one or more metal layers 1-240, which may be configured as a filter and/or may carry control signals from a control circuit configured to control transfer gates, as described further herein.

In some embodiments, pixel 1-112 may include one or more transfer gates configured to control operation of pixel 1-112 by applying an electrical bias to one or more semiconductor regions of pixel 1-112 in response to one or more control signals. For example, when transfer gate ST0 induces a first electrical bias at the semiconductor region between photodetection region PPD and storage region SD0, a transfer path (e.g., charge transfer channel) may be formed in the semiconductor region. Charge carriers (e.g., photo-electrons) generated in photodetection region PPD by the incident photons may flow along the transfer path to storage region SD0. In some embodiments, the first electrical bias may be applied during a collection period during which charge carriers from the sample are selectively directed to storage region SD0. Alternatively, when transfer gate ST0 provides a second electrical bias at the semiconductor region between photodetection region PPD and storage region SD0, charge carriers from photodetection region PPD may be blocked from reaching storage region SD0 along the transfer path. In some embodiments, drain gate REJ may provide a channel to drain D to draw noise charge carriers generated in photodetection region PPD by the excitation light away from photodetection region PPD and storage region SD0, such as during a rejection period before fluorescent emission photons from the sample reach photodetection region PPD. In some embodiments, during a readout period, transfer gate ST0 may provide the second electrical bias and transfer gate TX0 may provide an electrical bias to cause charge carriers stored in storage region SD0 to flow to the readout region, which may be a floating diffusion (FD) region, for processing.

It should be appreciated that, in accordance with various embodiments, transfer gates described herein may include semiconductor material(s) and/or metal, and may include a gate of a field effect transistor (FET), a base of a bipolar junction transistor (BJT), and/or the like.

In some embodiments, operation of pixel 1-112 may include one or more collection sequences, each collection sequence including one or more rejection (e.g., drain) periods and one or more collection periods. In one example, a collection sequence performed in accordance with one or more pulses of an excitation light source may begin with a rejection period, such as to discard charge carriers generated in pixel 1-112 (e.g., in photodetection region PD) responsive to excitation photons from the light source. For instance, the excitation photons may arrive at pixel 1-112 prior to the arrival of fluorescence emission photons from the sample well. Transfer gates for the charge storage regions may be biased to have low conductivity in the charge transfer channels coupling the charge storage regions to the photodetection region, blocking transfer and accumulation of charge carriers in the charge storage regions. A drain gate for the drain region may be biased to have high conductivity in a drain channel between the photodetection region and the drain region, facilitating draining of charge carriers from the photodetection region to the drain region. Transfer gates for any charge storage regions coupled to the photodetection region may be biased to have low conductivity between the photodetection region and the charge storage regions, such that charge carriers are not transferred to or accumulated in the charge storage regions during the rejection period.

Following the rejection period, a collection period may occur in which charge carriers generated responsive to the incident photons are transferred to one or more charge storage regions. During the collection period, the incident photons may include fluorescent emission photons, resulting in accumulation of fluorescent emission charge carriers in the charge storage region(s). For instance, a transfer gate for one of the charge storage regions may be biased to have high conductivity between the photodetection region and the charge storage region, facilitating accumulation of charge carriers in the charge storage region. Any drain gates coupled to the photodetection region may be biased to have low conductivity between the photodetection region and the drain region such that charge carriers are not discarded during the collection period.

Some embodiments may include multiple rejection and/or collection periods in a collection sequence, such as a second rejection period and second collection period following a first rejection period and a collection period, where each pair of rejection and collection periods is conducted in response to a pulse of excitation light. In one example, charge carriers generated in the photodetection region during each collection period of a collection sequence (e.g., in response to a plurality of pulses of excitation light) may be aggregated in a single charge storage region. In some embodiments, charge carriers aggregated in the charge storage region may be read out for processing prior to the next collection sequence. Alternatively or additionally, in some embodiments, charge carriers aggregated in a first charge storage region during a first collection sequence may be transferred to a second charge storage region sequentially coupled to the first charge storage region and read out simultaneously with the next collection sequence. In some embodiments, a processing circuit configured to read out charge carriers from one or more pixels may be configured to determine one or more of luminescence intensity information, luminescence lifetime information, luminescence spectral information, and/or any other mode of luminescence information associated with performing techniques described herein.

In some embodiments, a first collection sequence may include transferring, to a charge storage region at a first time following each excitation pulse, charge carriers generated in the photodetection response in response to the excitation pulse, and a second collection sequence may include transferring, to the charge storage region at a second time following each excitation pulse, charge carriers generated in the photodetection response in response to the excitation pulse. For example, the number of charge carriers aggregated after the first and second times may indicate luminance lifetime information of the received light.

As described further herein, pixels of an integrated device may be controlled to perform one or more collection sequences using one or more control signals from a control circuit of the integrated circuit, such as by providing the control signal(s) to drain and/or transfer gates of the pixel(s) of the integrated circuit. In some embodiments, charge carriers may be read out from the FD region of each pixel during a readout pixel associated with each pixel and/or a row or column of pixels for processing. In some embodiments, FD regions of the pixels may be read out using correlated double sampling (CDS) techniques.

### EXAMPLES

### Example 1. Barcode Recognition as a Means for Multiplexing

Single-molecule barcode recognition experiments were performed to investigate the potential for discriminating multiple different barcodes in a single reaction chamber. FIG. 7A illustrates a general process by which the experiments were carried out. As shown, a DNA barcode complex is immobilized to a reaction chamber surface through a streptavidin linkage group. The barcoded molecule includes a single-stranded DNA barcode region, a double-stranded region formed by a hybridized strand, and a bis-biotin moiety. The bis-biotin moiety of the barcoded molecule is bound by the streptavidin, which is further bound to biotin moieties on the surface. A labeled oligonucleotide probe containing a sequence complementary to the barcode is introduced, and hybridization of the probe to the immobilized DNA barcode is detected.

Also shown in FIG. 7A is a generic depiction of one of the labeled oligonucleotide probes used in these experiments. The labeled oligonucleotide probe (Tris-ATRho6G DNA) includes a single-stranded region complementary to the DNA barcode, and the single-stranded region is attached through a streptavidin linkage group to a double-stranded DNA having three copies of a fluorescent dye (ATTO Rho6G).

In a first set of experiments, two different labeled oligonucleotide probes were introduced to single reaction chambers having two different barcodes immobilized to the reaction chamber surface, and hybridization events between barcode and probe were monitored over a 24-hour period. FIG. 7B shows example single molecule intensity traces obtained during these experiments. As shown, a series of hybridization events give rise to a series of signal pulses detected in the intensity traces. Shown to the right of each intensity trace is a plot of intensity versus lifetime generated from the corresponding signal pulse data.

In a second set of experiments, three different labeled oligonucleotide probes were introduced to single reaction chambers having three different barcodes immobilized to the reaction chamber surface, and hybridization events between barcode and probe were monitored. FIG. 7C shows example single molecule intensity traces obtained during these experiments. Shown to the right of each intensity trace is a plot of intensity versus lifetime generated from the corresponding signal pulse data. FIG. 7D is a plot of intensity versus lifetime generated from a representative experiment using the three barcodes.

The intensity traces and plots shown in FIGs. 7B and 7C demonstrated that different barcodes can be distinguished based on differences in signal pulse patterns and/or luminescence properties (lifetime, intensity). For example, as shown in FIG. 7D, each of three different barcodes corresponds to a different cluster observed in a plot of intensity versus lifetime, allowing the three barcodes to be individually distinguishable. Thus, these experimental results confirmed that barcodes generate on-chip pulsing through DNA hybridization, and that barcodes display lifetime and kinetic pulse properties analogous to amino acid recognition during a dynamic peptide sequencing reaction, allowing similar analysis for barcode recognition and peptide sequencing.

### Example 2. Barcode Recognition and Polypeptide Sequencing

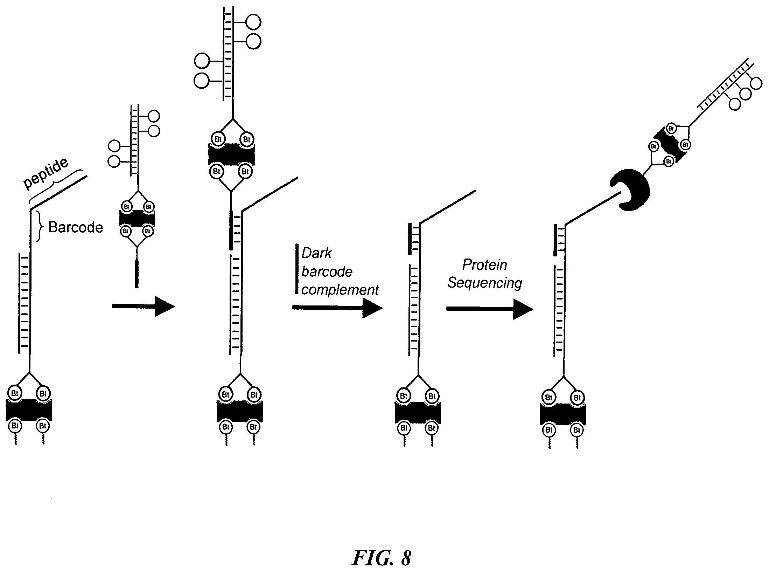

Single-molecule recognition experiments were performed to investigate the potential for performing barcode recognition and polypeptide sequencing in a single reaction chamber. FIG. 8 illustrates a general process by which barcode recognition is performed prior to polypeptide sequencing by amino acid recognition. As shown, a barcoded polypeptide is immobilized to a reaction chamber surface through a streptavidin linkage group, and barcode recognition is performed using a labeled oligonucleotide probe. Once barcode recognition is complete, the addition of an unlabeled oligonucleotide complementary to the barcode inhibits any further barcode recognition. This step is followed by polypeptide sequencing by amino acid recognition as described herein.

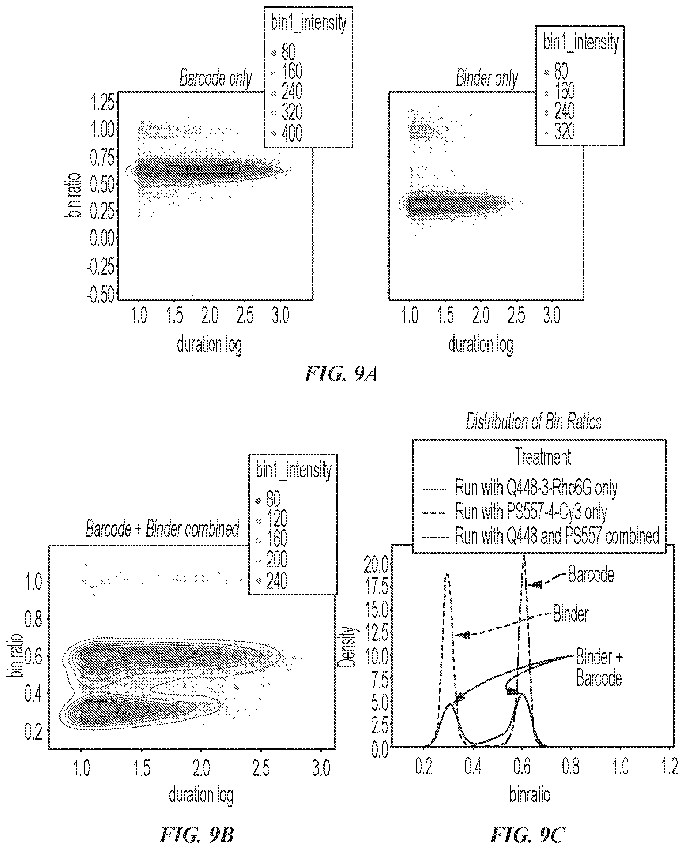

In these studies, a barcoded polypeptide was immobilized to the surface of single-molecule reaction chambers. In a first set of experiments, a labeled oligonucleotide probe complementary to the barcode was introduced to the reaction chambers, and barcode recognition data was obtained (FIG. 9A, left panel). The oligonucleotide probe was labeled with three copies of ATTO Rho6G dye. In a second set of experiments, a labeled terminal amino acid binding protein was introduced to the reaction chambers, and amino acid recognition data was obtained (FIG. 9A, right panel). The terminal amino acid binding protein was labeled with four copies of Cy3 dye. In a third set of experiments, the labeled oligonucleotide probe and the labeled amino acid binding protein were both introduced to the reaction chambers, and recognition data was obtained (FIG. 9B). A plot showing distribution of lifetime measurements (bin ratios) was generated using data from the three sets of experiments (FIG. 9C).

These experimental results confirmed that simultaneous recognition of barcode and amino acid can be observed for a barcoded polypeptide (FIG. 9B), and the lifetime and intensity data match the individual parameters (FIGs. 9A-9C). The results further showed that barcode and amino acid recognition can be performed sequentially according to the workflow shown in FIG. 8, or barcode and amino acid recognition can be performed simultaneously. In this example, the oligonucleotide probe and amino acid binding protein were labeled with different dye sets which allowed for lifetime differentiation.

Additional experiments were performed to investigate the potential for barcode and amino acid recognition under polypeptide degradation conditions used in polypeptide sequencing reactions.

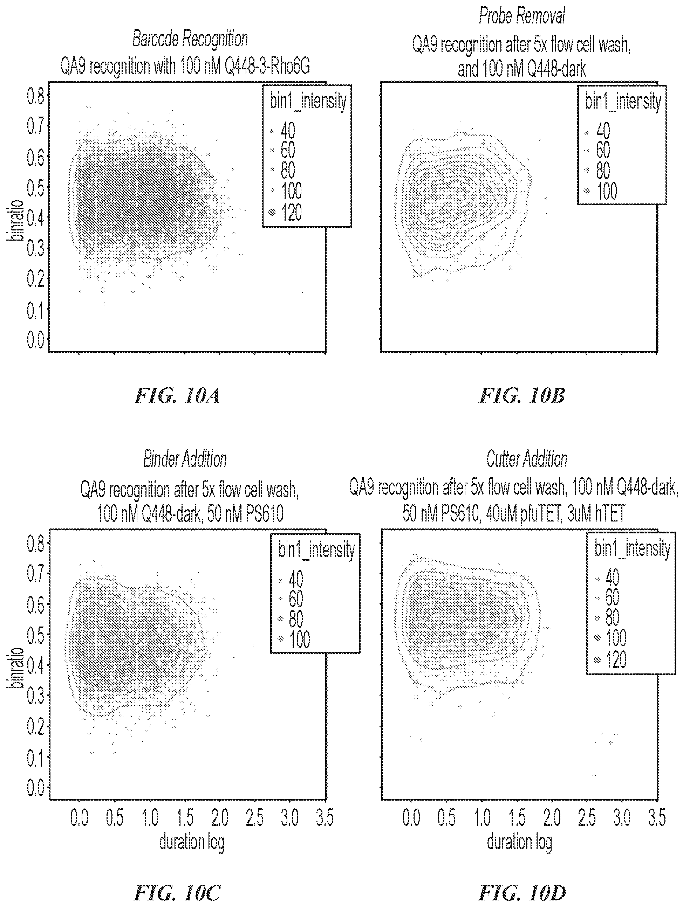

As in the previous studies, a barcoded polypeptide was immobilized to the surface of single-molecule reaction chambers. In accordance with the sequential workflow depicted in FIG. 8, a labeled oligonucleotide probe (100 nM) was introduced to the reaction chambers, and barcode recognition data was obtained (FIG. 10A). Next, an unlabeled oligonucleotide (100 nM) complementary to the barcode was introduced, which resulted in a decrease in detectable hybridization events between barcode and labeled probe (FIG. 10B). A labeled terminal amino acid binding protein (50 nM) was then introduced, and amino acid recognition data was obtained (FIG. 10C). Finally, cleaving reagent (40 µM PfuTET, 3 µM hTET) was introduced, which resulted in a decrease in detectable binding events between the labeled terminal amino acid binding protein and the polypeptide (FIG. 10D).

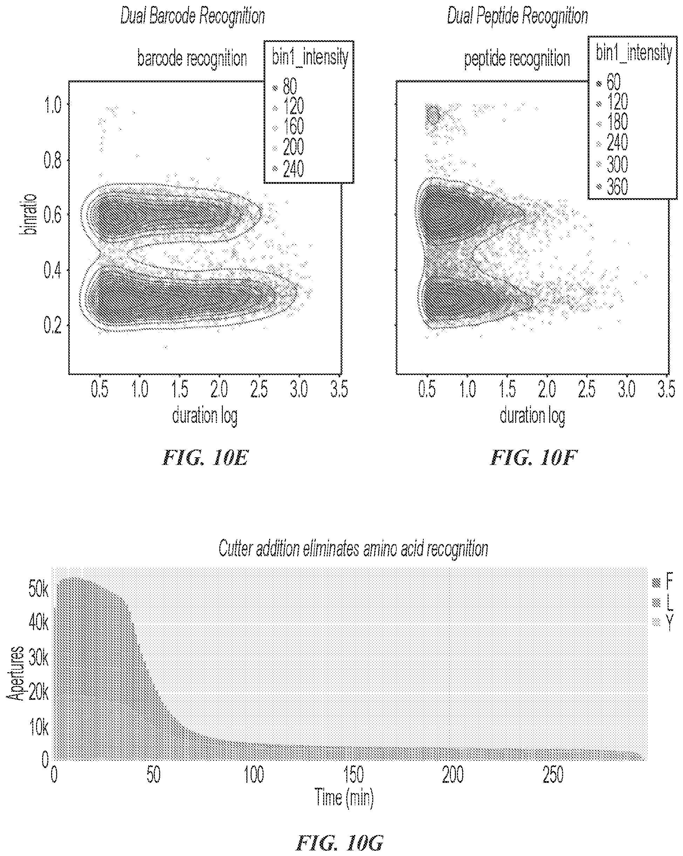

Similar experiments were performed using two different barcoded polypeptides immobilized to the surface of a single reaction chamber, where differently labeled oligonucleotide probes and differently labeled terminal amino acid binding proteins were introduced to reaction chambers. FIGs. 10E-10F show data confirming that the different barcodes (FIG. 10E) and terminal amino acids (FIG. 10F) of the two barcoded polypeptides were distinguishable by recognition. FIG. 10G shows that the addition of a cleaving reagent, which removes N-terminal amino acid, eliminates amino acid recognition.

These experimental results demonstrated the ability to recognize multiple different barcodes and polypeptides simultaneously using lifetime-differentiated oligonucleotide probes and amino acid binding proteins. Through the loss of signal after the addition of cutter, the results further demonstrated the ability of the barcoded polypeptide to be sequenced dynamically through the removal of N-terminal amino acids.

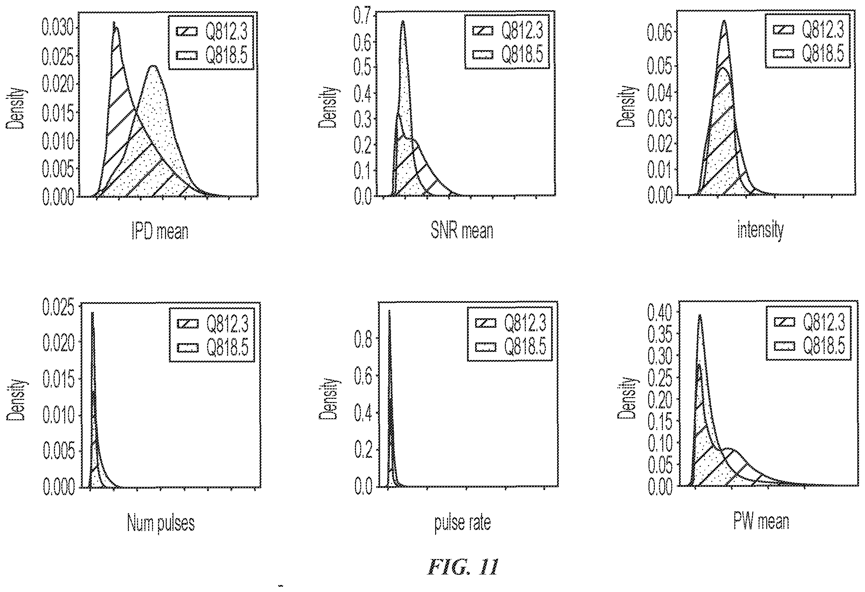

FIG. 11 shows data obtained in single molecule experiments involving barcode recognition of two different barcodes, illustrating that different kinetic pulse properties can be used to differentiate one barcode from another. In this example, two barcodes with unique sequences display different profiles of pulse duration, interpulse duration, and pulse SNR, which could be used to differentiate one from the other without the need to use dye sets with distinct lifetime (bin ratio) properties.

### Example 3. Barcode Readout via Hybridization

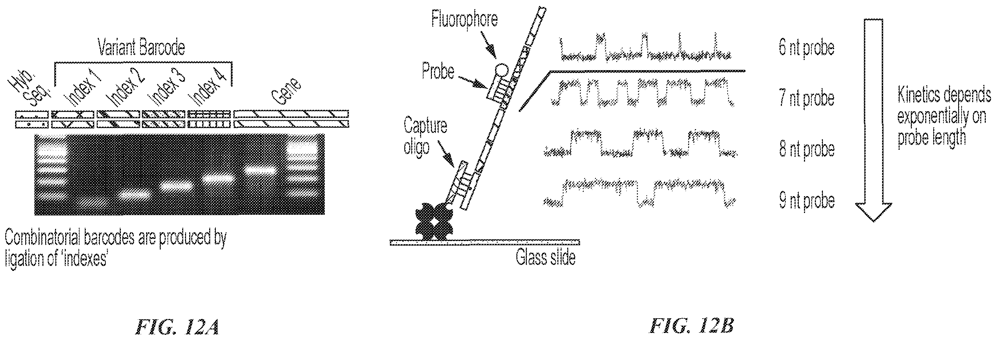

Combinatorial barcodes are produced by ligation of index sequences to produce a variant barcode with a hybridization sequence, as shown in FIG. 12A. The variant barcoded molecule is added to a streptavidin-coated slide, where the barcoded molecule is immobilized to the surface through the hybridization sequence which binds to a capture oligonucleotide attached to streptavidin (FIG. 12B). The immobilized barcoded molecule is contacted with a labeled oligonucleotide probe that binds to the index sequences, and these binding events are detected as a series of signal pulses. Also shown in 12B, the pattern in the observed signal pulse will vary depending on oligonucleotide probe length.

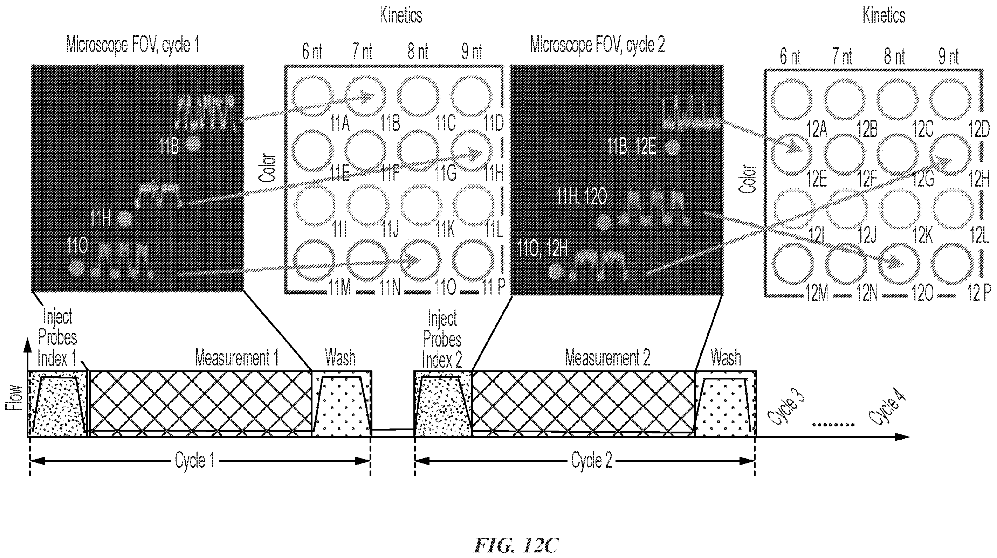

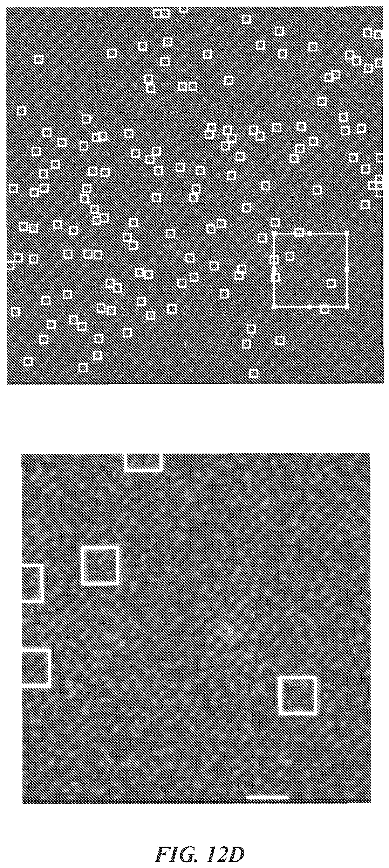

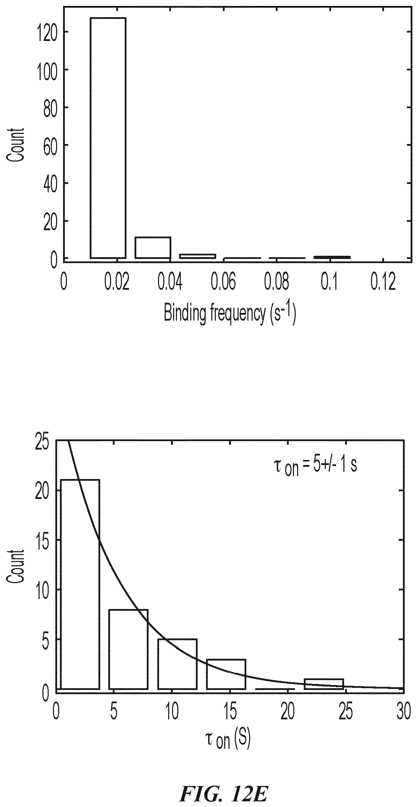

FIG. 12C shows an example workflow for a barcode recognition assay, which involves iterative steps of washing in different oligonucleotide probes at different points over the course of the assay. By distinguishing probes based both on color and kinetics in this approach, it is possible to have 16 sequences per index, or 65,536 variants. FIG. 12D shows on-chip imaging of recognition assays performed in parallel (region highlighted in top image shown zoomed in bottom image). FIG. 12E shows plots evaluating binding frequency (top) and τₒₙ (bottom).

### Example 4. Single-Molecule Screening

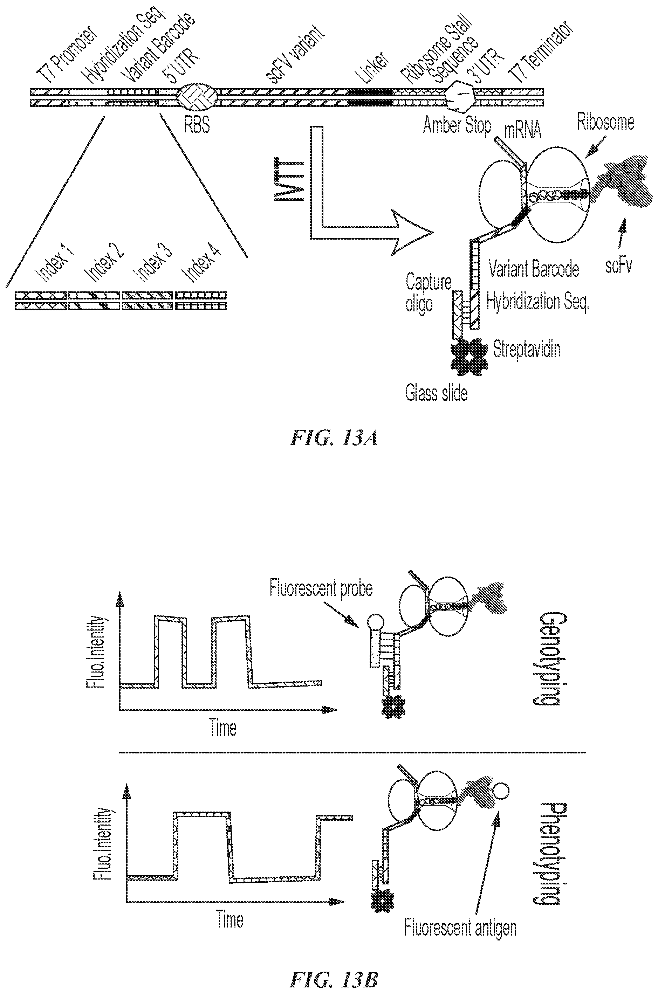

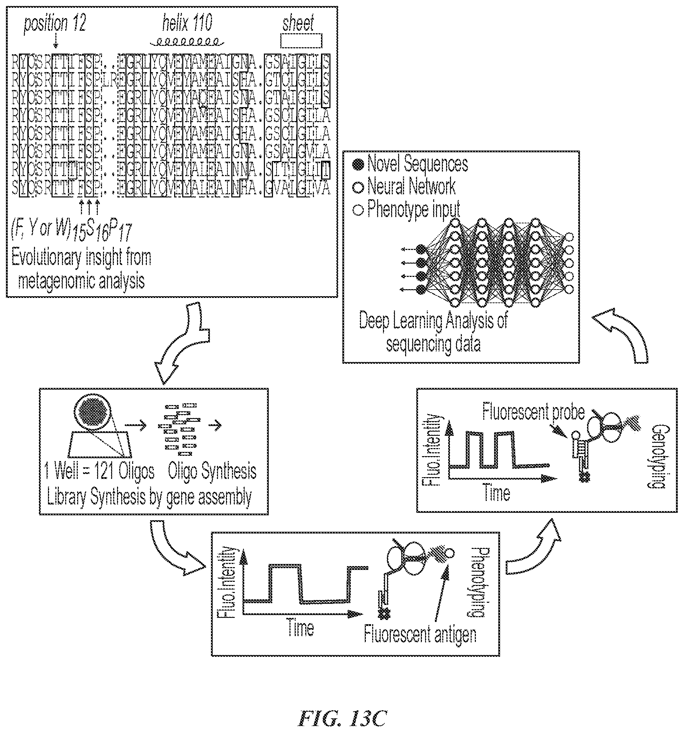

Single-molecule screening techniques fill the gap between ultra-high throughput selections and low throughput secondary screens, offering a middle ground with high throughput (10⁵ on a nanophotonic chip) and precise phenotypic characterization. Variant barcodes are included in a coding construct used in an antibody screening assay in which the resulting product from *in vitro* transcription/translation contains the variant barcodes for the analysis (FIG. 13A). As shown in FIG. 13B, fluorescent probes are used to interrogate the variant barcodes using binding kinetics (top), and the antibody/antigen screening is similarly based on single-molecule kinetics (bottom). FIG. 13C shows an example workflow for a directed evolution screening approach.

### EQUIVALENTS AND SCOPE

In the claims articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention includes embodiments in which more than one, or all of the group members are present in, employed in, or otherwise relevant to a given product or process.

Furthermore, the invention encompasses all variations, combinations, and permutations in which one or more limitations, elements, clauses, and descriptive terms from one or more of the listed claims is introduced into another claim. For example, any claim that is dependent on another claim can be modified to include one or more limitations found in any other claim that is dependent on the same base claim. Where elements are presented as lists, e.g., in Markush group format, each subgroup of the elements is also disclosed, and any element(s) can be removed from the group. It should be understood that, in general, where the invention, or aspects of the invention, is/are referred to as comprising particular elements and/or features, certain embodiments of the invention or aspects of the invention consist, or consist essentially of, such elements and/or features. For purposes of simplicity, those embodiments have not been specifically set forth *in haec verba* herein.

The phrase "and/or," as used herein in the specification and in the claims, should be understood to mean "either or both" of the elements so conjoined, *i.e.*, elements that are conjunctively present in some cases and disjunctively present in other cases. Multiple elements listed with "and/or" should be construed in the same fashion, *i.e.,* "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, *i.e.,* the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (*i.e.* "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, *i.e.,* to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03. It should be appreciated that embodiments described in this document using an open-ended transitional phrase (e.g., "comprising") are also contemplated, in alternative embodiments, as "consisting of" and "consisting essentially of" the feature described by the open-ended transitional phrase. For example, if the application describes "a composition comprising A and B," the application also contemplates the alternative embodiments "a composition consisting of A and B" and "a composition consisting essentially of A and B."

Where ranges are given, endpoints are included. Furthermore, unless otherwise indicated or otherwise evident from the context and understanding of one of ordinary skill in the art, values that are expressed as ranges can assume any specific value or sub-range within the stated ranges in different embodiments of the invention, to the tenth of the unit of the lower limit of the range, unless the context clearly dictates otherwise.

This application refers to various issued patents, published patent applications, journal articles, and other publications. If there is a conflict between any of the references and the instant specification, the specification shall control. In addition, any particular embodiment of the present invention that falls within the prior art may be explicitly excluded from any one or more of the claims. Because such embodiments are deemed to be known to one of ordinary skill in the art, they may be excluded even if the exclusion is not set forth explicitly herein. Any particular embodiment of the invention can be excluded from any claim, for any reason, whether or not related to the existence of prior art.

The recitation of a listing of chemical groups in any definition of a variable herein includes definitions of that variable as any single group or combination of listed groups. The recitation of an embodiment for a variable herein includes that embodiment as any single embodiment or in combination with any other embodiments or portions thereof. The recitation of an embodiment herein includes that embodiment as any single embodiment or in combination with any other embodiments or portions thereof.

## Claims

1. A method comprising:
contacting a molecular barcode with a barcode recognition molecule that binds to one or more sites on the molecular barcode, wherein the molecular barcode is attached to an analyte comprising a polypeptide;
detecting a series of signal pulses indicative of binding interactions between the barcode recognition molecule and the molecular barcode;
determining the identity of the molecular barcode based on a barcode-specific pattern in the series of signal pulses; and
sequencing the polypeptide.

2. The method of claim 1, wherein the analyte is immobilized to a surface through the molecular barcode.

3. The method of claim 2, wherein the molecular barcode is immobilized to the surface through a linkage group comprising at least one biomolecule.

4. The method of claim 3, wherein the linkage group comprises a double-stranded nucleic acid and/or a protein-ligand complex comprising an avidin protein bound to at least one bisbiotin moiety.

5. The method of any one of claims 1-4, wherein the molecular barcode is a nucleic acid barcode.

6. The method of any one of claims 1-4, wherein the molecular barcode is a polypeptide barcode.

7. The method of any one of claims 1-6, wherein the barcode recognition molecule is an oligonucleotide.

8. The method of any one of claims 1-6, wherein the barcode recognition molecule is a protein or a nucleic acid aptamer.

9. The method of any one of claims 1-8, wherein the barcode recognition molecule comprises at least one detectable label.

10. The method of claim 9, wherein the barcode recognition molecule is attached to a labeled biomolecule comprising the at least one detectable label.

11. The method of claim 10, wherein the barcode recognition molecule is attached to the labeled biomolecule through a linkage group comprising at least one biomolecule.

12. The method of any one of claims 1-11, wherein the contacting comprises contacting the molecular barcode with two or more barcode recognition molecules that bind to different or overlapping sites on the molecular barcode.

13. The method of claim 12, wherein the two or more barcode recognition molecules are simultaneously contacted with the molecular barcode in the same mixture.

14. The method of any one of claims 1-13, wherein sequencing the polypeptide comprises:
contacting the polypeptide with one or more terminal amino acid recognition molecules; and
detecting a series of signal pulses indicative of association of the one or more terminal amino acid recognition molecules with successive amino acids exposed at a terminus of the polypeptide while the polypeptide is being degraded, thereby sequencing the polypeptide.

15. The method of any one of claims 1-14, wherein the method is performed in a single reaction vessel.

## Patentansprüche

1. Verfahren, umfassend:
Inkontaktbringen eines molekularen Barcodes mit einem Barcode-Erkennungsmolekül, das an eine oder mehrere Stellen auf dem molekularen Barcode bindet, wobei der molekulare Barcode an einen Analyten angeheftet ist, der ein Polypeptid umfasst;
Detektieren einer Reihe von Signalimpulsen, die Bindungswechselwirkungen zwischen dem Barcode-Erkennungsmolekül und dem molekularen Barcode anzeigen; Bestimmen der Identität des molekularen Barcodes basierend auf einem Barcode-spezifischen Muster in der Reihe von Signalimpulsen; und
Sequenzierung des Polypeptids.

2. Verfahren nach Anspruch 1, wobei der Analyt durch den molekularen Barcode an einer Oberfläche immobilisiert ist.

3. Verfahren nach Anspruch 2, wobei der molekulare Barcode durch eine Verknüpfungsgruppe, die mindestens ein Biomolekül umfasst, an der Oberfläche immobilisiert ist.

4. Verfahren nach Anspruch 3, wobei die Verknüpfungsgruppe eine doppelsträngige Nukleinsäure und/oder einen Protein-Ligand-Komplex umfasst, der ein Avidin-Protein umfasst, das an mindestens eine Bis-Biotin-Einheit gebunden ist.

5. Verfahren nach einem der Ansprüche 1-4, wobei der molekulare Barcode ein Nukleinsäurebarcode ist.

6. Verfahren nach einem der Ansprüche 1-4, wobei der molekulare Barcode ein Polypeptidbarcode ist.

7. Verfahren nach einem der Ansprüche 1-6, wobei das Barcode-Erkennungsmolekül ein Oligonukleotid ist.

8. Verfahren nach einem der Ansprüche 1-6, wobei das Barcode-Erkennungsmolekül ein Protein oder ein Nukleinsäure-Aptamer ist.

9. Verfahren nach einem der Ansprüche 1-8, wobei das Barcode-Erkennungsmolekül mindestens eine nachweisbare Markierung umfasst.

10. Verfahren nach Anspruch 9, wobei das Barcode-Erkennungsmolekül an ein markiertes Biomolekül angeheftet ist, das die mindestens eine nachweisbare Markierung umfasst.

11. Verfahren nach Anspruch 10, wobei das Barcode-Erkennungsmolekül über eine Verknüpfungsgruppe, die mindestens ein Biomolekül umfasst, an das markierte Biomolekül angeheftet ist.

12. Verfahren nach einem der Ansprüche 1-11, wobei das Inkontaktbringen das Inkontaktbringen des molekularen Barcodes mit zwei oder mehreren Barcode-Erkennungsmolekülen umfasst, die an verschiedene oder überlappende Stellen auf dem molekularen Barcode binden.

13. Verfahren nach Anspruch 12, wobei die zwei oder die mehreren Barcode-Erkennungsmoleküle gleichzeitig mit dem molekularen Barcode in derselben Mischung in Kontakt gebracht werden.

14. Verfahren nach einem der Ansprüche 1-13, wobei die Sequenzierung des Polypeptids umfasst:
Inkontaktbringen des Polypeptids mit einem oder mehreren terminale-Aminosäure-Erkennungsmolekülen; und
Detektieren einer Reihe von Signalimpulsen, die eine Assoziation des einen oder der mehreren terminale-Aminosäure-Erkennungsmoleküle mit aufeinanderfolgenden Aminosäuren anzeigen, die an einem Terminus des Polypeptids exponiert werden, während das Polypeptid abgebaut wird, wodurch das Polypeptid sequenziert wird.

15. Verfahren nach einem der Ansprüche 1-14, wobei das Verfahren in einem einzigen Reaktionsgefäß durchgeführt wird.

## Revendications

1. Procédé comprenant :
la mise en contact d'un code à barres moléculaire avec une molécule de reconnaissance de code à barres qui se lie à un ou plusieurs sites sur le code à barres moléculaire, le code à barres moléculaire étant fixé à un analyte comprenant un polypeptide ;
la détection d'une série d'impulsions de signal indiquant des interactions de liaison entre la molécule de reconnaissance de code à barres et le code à barres moléculaire ;
la détermination de l'identité du code à barres moléculaire en fonction d'un motif spécifique au code à barres dans la série d'impulsions de signal ; et
le séquençage du polypeptide.

2. Procédé selon la revendication 1, dans lequel l'analyte est immobilisé sur une surface par l'intermédiaire du code à barres moléculaire.

3. Procédé selon la revendication 2, dans lequel le code à barres moléculaire est immobilisé sur la surface par l'intermédiaire d'un groupe de liaison comprenant au moins une biomolécule.

4. Procédé selon la revendication 3, dans lequel le groupe de liaison comprend un acide nucléique double brin et/ou un complexe protéine-ligand comprenant une protéine avidine liée à au moins un fragment bis-biotine.

5. Procédé selon l'une quelconque des revendications 1 à 4, dans lequel le code à barres moléculaire est un code à barres acide nucléique.

6. Procédé selon l'une quelconque des revendications 1 à 4, dans lequel le code à barres moléculaire est un code à barres polypeptidique.

7. Procédé selon l'une quelconque des revendications 1 à 6, dans lequel la molécule de reconnaissance de code à barres est un oligonucléotide.

8. Procédé selon l'une quelconque des revendications 1 à 6, dans lequel la molécule de reconnaissance de code à barres est une protéine ou un aptamère d'acide nucléique.

9. Procédé selon l'une quelconque des revendications 1 à 8, dans lequel la molécule de reconnaissance de code à barres comprend au moins un marqueur détectable.

10. Procédé selon la revendication 9, dans lequel la molécule de reconnaissance de code à barres est fixée à une biomolécule marquée comprenant l'au moins un marqueur détectable.

11. Procédé selon la revendication 10, dans lequel la molécule de reconnaissance de code à barres est fixée à la biomolécule marquée par l'intermédiaire d'un groupe de liaison comprenant au moins une biomolécule.

12. Procédé selon l'une quelconque des revendications 1 à 11, dans lequel la mise en contact comprend la mise en contact du code à barres moléculaire avec deux ou plus de deux molécules de reconnaissance de code à barres qui se lient à des sites différents ou se chevauchant sur le code à barres moléculaire.

13. Procédé selon la revendication 12, dans lequel les deux ou plus de deux molécules de reconnaissance de code à barres sont simultanément mises en contact avec le code à barres moléculaire dans le même mélange.

14. Procédé selon l'une quelconque des revendications 1 à 13, dans lequel le séquençage du polypeptide comprend :
la mise en contact du polypeptide avec une ou plusieurs molécules de reconnaissance d'acides aminés terminaux ; et
la détection d'une série d'impulsions de signal indiquant l'association de la ou des molécules de reconnaissance d'acides aminés terminaux avec des acides aminés successifs exposés à une extrémité du polypeptide pendant que le polypeptide est dégradé, ce qui permet le séquençage du polypeptide.

15. Procédé selon l'une quelconque des revendications 1 à 14, le procédé étant mis en œuvre dans un seul récipient de réaction.
